# EUROPEAN PATENT SPECIFICATION

(11) **EP 2 956 471 B1**
(45) Date of publication and mention of the grant of the patent: **10.04.2024**
(21) Application number: 13875211.8
(22) Date of filing: 15.02.2013
(51) Int. Cl.: A61P 9/10, A61P 11/00, A61P 17/00, A61P 9/06, A61P 19/02, C07K 14/715, A61P 3/10, A61K 38/17, A61P 19/06, A61P 27/02, A61P 27/04, A61P 27/14, A61P 43/00, A61P 29/00

(54) **IL-1BETA INHIBITOR COMPOSITION AND USE THEREOF**
IL-1BETA-INHIBITORZUSAMMENSETZUNG UND VERWENDUNG DAVON
COMPOSITION D'INHIBITEUR D'IL-1BETA ET UTILISATION CORRESPONDANTE

(43) Date of publication of application: 23.12.2015
(60) Divisional application: 21208571.6
(73) Proprietor: R-Pharm International, LLC, 123154 Moscow (RU)
(72) Inventor: LAVROVSKY, Yan, San Diego, CA 92121 (US); XU, Ting, Sudbury, MA 01776 (US); REPIK, Alexey, Moscow 121609 (RU); XU, Tao, Suzhou Jiangsu Province 215125 (CN); IGNATIEV, Vasily, Moscow 121353 (RU); SAMSONOV, Mikhail, Moscow 125313 (RU)
(74) Representative: Osmans, Voldemars
(86) International application number: PCT/US2013/026349
(87) International publication number: WO 2014/126582

(56) References cited:
- WO-A1-2009/089004
- WO-A1-2014/035361
- US-B2- 7 666 622
- US-B2- 7 666 622
- ECONOMIDES A N ET AL: "Cytokine traps: multi-component, high-affinity blockers of cytokine action", NATURE MEDICINE, NATURE PUB. CO, NEW YORK, vol. 9, no. 1, 1 January 2003 (2003-01-01) , pages 47-52, XP002256034, ISSN: 1078-8956, DOI: 10.1038/NM811
- ECONOMIDES ARIS N. ET AL.: 'Cytokine traps: multi-component, high-affinity blockers of cytokine action.' NATURE MEDICINE vol. 9, no. 1, 2003, pages 47 - 52, XP002256034
- DATABASE PUBMED [Online] 01 January 2011 '[CAPS: CRYOPYRIN-ASSOCIATED PERIODIC SYNDROME].', XP055279021 Retrieved from NCBI Database accession no. 2204124 & SAITO MK .: 'CAPS: cryopyrin-associated periodic syndrome.' NIHON RINSHO MENEKI GAKKAI KAISHI vol. 34, no. 5, 2011, pages 369 - 377, XP055279021
- DATABASE PUBMED [Online] 01 January 2010 ' ROLE OF IL-1BETA IN TYPE 2 DIABETES.', XP055290630 Retrieved from NCBI Database accession no. 20588114 & DINARELLO CA ET AL.: 'Role of IL -1 beta in type 2 diabetes' CURR OPIN ENDOCRINOL DIABETES OBES vol. 17, no. 4, 2010, pages 314 - 321, XP009136506
- BHASKAR V ET AL.: 'Monoclonal antibodies targeting IL -1 beta reduce biomarkers of atherosclerosis in vitro and inhibit atherosclerotic plaque formation in Apolipoprotein E-deficient mice.' ATHEROSCLEROSIS vol. 216, no. 2, 2011, pages 313 - 320, XP028226561
- DATABASE PUBMED [Online] 28 October 2009 'ANALYSIS OF INFLAMMATORY CYTOKINES IN THE TEARS OF DRY EYE PATIENTS', XP055279023 Retrieved from NCBI Database accession no. 19724208 & MASSINGALE ML ET AL.: 'Analysis of inflammatory cytokines in the tears of dry e patients.' CORNEA vol. 28, no. 9, 2009, pages 1023 - 1027

## Description

### FIELD OF THE INVENTION

Generally, the invention relates to the field of biological pharmaceuticals as well as their use in conditions associated with inflammatory disorders (e.g. rheumatoid arthritis, Crohn's disease, etc.), diabetes, cardiovascular disease and gout. More specifically, the invention relates to a heterodimeric IL-1R1/IL-1RAcP -derived composition that is capable of inhibiting IL-1β cytokine.

### BACKGROUND

The interleukin-1 (IL-1) family of cytokines comprises 11 proteins (IL-1F1 to IL-1F11) encoded by 11 distinct genes in humans and mice. IL-1-type cytokines are major mediators of innate immune reactions, and blockade of the founding members IL-1 or IL-1β by the interleukin-1 receptor antagonist (IL-1RA) has demonstrated a central role of IL-1 in a number of human autoinflammatory diseases. IL-1 or IL-1β rapidly increase messenger RNA expression of hundreds of genes in multiple different cell types. The potent proinflammatory activities of IL-1and IL-1β are restricted at three major levels: (i) synthesis and release, (ii) membrane receptors, and (iii) intracellular signal transduction. This pathway summarizes extracellular and intracellular signaling of IL-1 or IL-1β, including positive- and negative-feedback mechanisms that amplify or terminate the IL-1 response. In response to ligand binding of the receptor, a complex sequence of combinatorial phosphorylation and ubiquitination events results in activation of nuclear factor kappa-B signaling and the JNK and p38 mitogen-activated protein kinase pathways, which, cooperatively, induce the expression of canonical IL-1 target genes (such as IL-6, IL-8, MCP-1, COX-2, IB, IL-1, IL-1β, MKP-1) by transcriptional and posttranscriptional mechanisms. Of note, most intracellular components that participate in the cellular response to IL-1 also mediate responses to other cytokines (IL-18 and IL-33), Toll-like-receptors (TLRs), and many forms of cytotoxic stresses (see Weber A., et al., Sci Signal., 2010 Jan 19;3(105)).

IL-1 and IL-1β independently bind the type I IL-1 receptor (IL-1R1), which is ubiquitously expressed. A third specific ligand, the IL-1 receptor antagonist (IL-1RA), binds the IL-1RI with similar specificity and affinity but does not activate the receptor and trigger downstream signaling. The IL-1 receptor accessory protein (IL-1RAcP) serves as a co-receptor that is required for signal transduction of IL-1/IL-1RI complexes, and this co-receptor is also necessary for activation of IL-1R1 by other IL-1 family members, in particular IL-18 and IL-33. The type II IL-1 receptor (IL-1R2) binds IL-1 and IL-1β but lacks a signaling-competent cytosolic part and thus serves as a decoy receptor. The II,-1RA, the plasma membrane-anchored IL-1R2, and the naturally occurring "shed" domains of each of the extracellular IL-1 receptor chains (termed sIL-1RI, sIL-1RII, and sIL-1RAcP, where "s" stands for soluble) provide inducible negative regulators of IL-1 signaling in the extracellular space whose abundance, which is regulated by a combination of increased transcription and controlled release, can limit or terminate IL-1 effects.

The initial step in IL-1 signal transduction is a ligand-induced conformational change in the first extracellular domain of the IL-1RI that facilitates recruitment of IL-1RacP. Through conserved cytosolic regions called Toll- and IL-1R-like (TIR) domains, the trimeric complex rapidly assembles two intracellular signaling proteins, myeloid differentiation primary response gene 88 (MYD88) and interleukin-1 receptor-activated protein kinase (IRAK) 4. Mice lacking MYD88 or IRAK4 show severe defects in IL-1 signaling. Similarly, humans with mutations in the IRAK4 gene have defects in IL-1RI and Toll-like receptor (TLR) signaling. IL-1, IL-1RI, IL-RAcP, MYD88, and IRAK4 form a stable IL-1-induced first signaling module. This is paralleled by the (auto)phosphorylation of IRAK4, which subsequently phosphorylates IRAK1 and IRAK2, and then this is followed by the recruitment and oligomerization of tumor necrosis factor-associated factor (TRAF) 6. IRAK1 and 2 function as both adaptors and protein kinases to transmit downstream signals. Complexes of IRAK1, IRAK2, and TRAF6 dissociate from the initial receptor complex, and cells lacking these proteins have impaired activation of the transcription factors nuclear factor kappa-B (NF-kappa-B) and activator protein 1 (AP-1).

Overproduction of IL-1 is the cause of many inflammatory disorders. For example, IL-1 has been linked to the pathology of diabetes, cardiovascular disease, gout and certain types of arthritis (e.g. rheumatoid arthritis (RA)).

Rilonacept is an IL-1 antagonist which includes an IL-1-specific fusion protein which comprises an IL-1 binding portion of the extracellular domain of human IL1-RAcP, an IL-1 binding portion of the extracellular domain of human IL-1RI, and a multimerizing component. This IL-1-specific fusion protein is described in U.S. Pat. No. 6,472,179, U.S. patent publication No. 2003/0143697, published 31 Jul. 2003, U.S. Pat. No. 7,361,350, and U.S. patent publication No. 2005/0197293, published 8 Sep. 2005.

Rilonacept under the trade name ARCALYST was approved by U.S. Food and Drug Administration (FDA) for the treatment of Cryopyrin-Associated Periodic Syndromes (CAPS), including Familial Cold Auto-inflammatory Syndrome (FCAS) and Muckle-Wells Syndrome (MWS) in adults and children 12 and older. Further clinical trials of rilonacept are currently under way, i.e. for gout.

The article ECONOMIDES A.N. et al., "Cytokine traps: multi-component, high-affinity blockers of cytokine action", NATURE MEDICINE, NATURE PUB. CO, NEW YORK, vol. 9, no. 1, (2003-01-01), pages 47-52, XP002256034, discloses the engineering of separate constructs encoding the extracellular domain of IL6Ra or gp130 fused to the Fc portion of human immunoglobulin 1. The article also report the design of a second generation of cytokine traps, wherein the extracellular domains of two distinct receptors are fused in line and then fused to Fc portion of human LgG1.

### SUMMARY OF THE INVENTION

In certain aspects, the present invention provides for a heterodimeric protein composition capable of binding human IL-1β (GenBank: AAH08678.1). The protein composition comprises a first polypeptide which includes a first amino acid sequence which contains amino acids 18 through 333 of human IL1-R1 (GenBank: AAM88423.1), and a second amino acid sequence which contains a first mutant of a Fc portion of human immunoglobulin gamma-1 Fc (GenBank: J00228.1). The protein composition also comprises a second polypeptide which includes another first amino acid sequence containing amino acids 21 through 358 of human II,1-RAcP (GenBank: BAA25421.1), and another second amino acid sequence which contains a second mutant of the Fc portion of human immunoglobulin gamma-1 Fc. In the protein composition, the first and second mutants are selected as to favor heterodimeric assembly between the first and second mutants over any homodimeric assembly. In the protein composition, the first polypeptide comprises the amino acid sequence of SEQ ID N°1 and the second polypeptide comprises the amino acid sequence of SEQ ID N°2. The protein composition may be capable of exhibiting human IL-1β binding activity in an ELISA assay with an EC50 of about 50 ng/ml.

In certain aspects, the present invention provides for a therapeutic composition. The therapeutic composition comprises a heterodimeric protein composition capable of binding human IL-1β. The protein composition comprises a first polypeptide which includes a first amino acid sequence which contains amino acids 18 through 333 of human IL1-R1, and a second amino acid sequence which contains a first mutant of the Fc portion of human immunoglobulin gamma-1 Fc. The protein composition also comprises a second polypeptide which includes another first amino acid sequence containing amino acids 21 through 358 of human IL1-RAcP, and another second amino acid sequence which contains a second mutant of the Fc portion of human immunoglobulin gamma-1 Fc. In the protein composition, the first and second mutants are selected as to favor heterodimeric assembly between the first and second mutants over any homodimeric assembly.

In the protein composition, the first polypeptide comprises the amino acid sequence of SEQ ID N°1 and the second polypeptide comprises the amino acid sequence of SEQ ID N°2.

The therapeutic composition may exhibit a half-life of the heterodimeric protein composition in systemic circulation in mice after a subcutaneous administration at a dose of 5 mg/kg of at least about 88 hours, as assayed by human Fc ELISA.

In certain aspects, the present invention provides for an isolated nucleic acid encoding a polypeptide comprising an amino acid sequence of any one of SEQ ID N°1, SEQ ID N°2, SEQ ID N°3 and SEQ ID N°5.

The codon usage of the nucleic acid may be optimized for high expression of the polypeptide in a mammalian cell.

In certain aspects, the present invention provides for an isolated nucleic acid encoding a polypeptide comprising amino acid sequence of SEQ ID N°3 and comprising the sequence of SEQ ID N°4, or an isolated nucleic acid sequence encoding a polypeptide comprising amino acid sequence of SEQ ID N°5 and comprising the sequence of SEQ ID N°6.

The nucleic acid may comprise an expression vector.

In certain aspects, the present invention provides for an isolated nucleic acid of SEQ ID NO. 7.

In certain aspects, the present invention provides for a heterologous expression system. The expression system harbors an expression vector comprising a nucleic acid sequence encoding a first polypeptide containing amino acid sequence of SEQ ID NO. 3 and another nucleic acid sequence encoding a second polypeptide containing amino acid sequence of SEQ ID NO. 4. The expression vector of the expression system may be harbored in a mammalian cell. The mammalian cell may be a CHO cell. The expression system may be capable of expressing a heterodimeric protein comprising a first polypeptide containing amino acid sequence of SEQ ID NO. 1 and a second polypeptide containing amino acid sequence of SEQ ID NO. 2. The level of expression of the heterodimeric protein may be at least 300 mg per liter of cell culture.

In certain aspects, the present invention provides a substance for use in the treatment or prevention of a disease associated with modulation of activity of human IL-1β. The substance comprises a heterodimeric protein comprised of a first polypeptide containing amino acid sequence of SEQ ID NO. 1 and a second polypeptide containing amino acid sequence of SEQ ID NO. 2. The disease associated with modulation of activity of human IL-1β may be an arthritis, a gout, a rheumatoid arthritis, a Cryopyrin-Associated Periodic Syndromes (CAPS), a scleroderma, a diabetes, a atherosclerosis, a dry eye disease, an ocular allergy, or an uveitis.

In certain aspects the present invention provides for a heterodimeric protein composition capable of binding human, wherein said heterodimeric composition comprises a first polypeptide comprising the SEQ ID N°1 and a second polypeptide comprising the amino acid sequence of SEQ ID N °2. The protein composition is capable of inhibiting human IL-6 production in human lung fibroblasts in response to treating the fibroblasts with human IL-1β. The inhibiting is characterized by an IC50 value of about 2.3 pM.

### BRIEF DESCRIPTION OF THE DRAWINGS

The following drawings and descriptions are provided to aid in the understanding of the invention:
**Figure 1** illustratively shows a heterodimeric protein assembly of the present teachings comprising an extracellular portion of IL1-R1 fused with an IgG-Fc domain (Fc-II) via a flexible linker and an extracellular portion of IL1-RAcP fused with another IgG-Fc domain (Fc-V) via another flexible linker;
**Figure 2** schematically shows the map of PKN012 plasmid and annotated sequence used in the cloning of the polypeptides of the present invention;
**Figure 3** shows representative transfection growth curve obtained in the process of generating stable cell lines for expressing the polypeptides of the present invention;
**Figure 4** shown a size-exclusion HPLC analytical chromatogram of the sample containing heterodimers comprising polypeptide of SEQ ID NO. 1 and polypeptide of SEQ ID NO. 2 after the anion exchange chromatography purification step;
**Figure 5** shown a SDS-PAGE analysis of the sample containing heterodimers comprising polypeptide of SEQ ID NO. 1 and polypeptide of SEQ ID NO. 2 after the anion exchange chromatography purification step;
**Figure 6** shows a typical binding curve of a purified sample containing heterodimers comprising polypeptide of SEQ ID NO. 1 and polypeptide of SEQ ID NO. 2 in an ELISA assay using commercially available human IL-1β;
**Figure 7** shows the calibration curve obtained in the IL-1 antibody mediated inhibition of IL-6 production assay experiment;
**Figure 8** shows the results of IL-1β-mediated IL-6 production and IL-6 recovery from the culture medium where MRC5 cells were incubated for 24 hours with IL-1β at a final concentration of 50 pg/ml (IL-1 alone) or left untreated (Cells Ctrl) while culture medium not exposed to the cells was spiked with IL-6 at a final concentration of 20 pg/ml to estimate IL-6 recovery;
**Figure 9** shows the results of the measurements of IL-1β-mediated IL-6 production and IL-6 recovery from the culture medium where MRC5 cells were incubated for 24 hours with IL-1β at a final concentration of 50 pg/ml (IL-1 alone) or left untreated (Cells Ctrl). Culture medium not exposed to the cells was spiked with IL-6 at a final concentration of 20 pg/ml to estimate IL-6 recovery;
**Figure 10** shows the calibration curve obtained in the inhibition of IL-6 production assay conducted with IL1R-FcV-RAcP-FcII heterodimer of the present teachings;

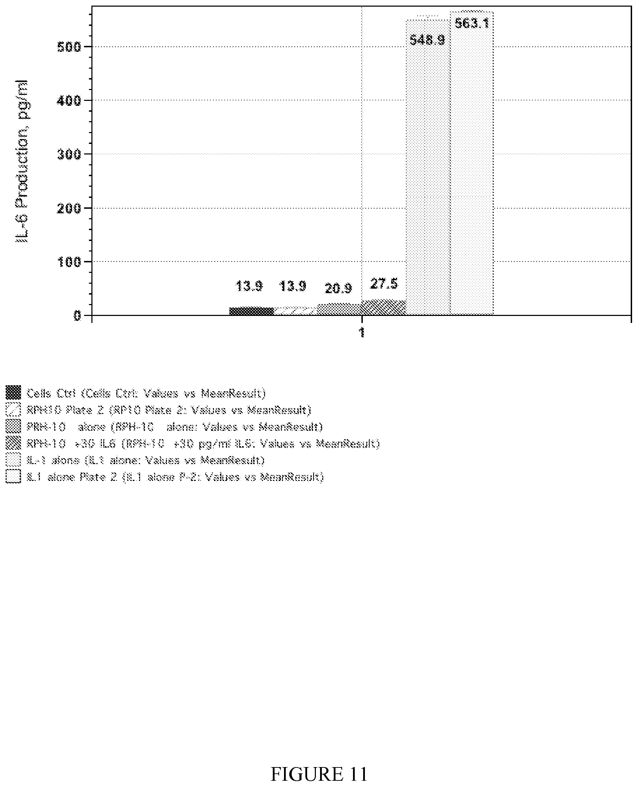

**Figure 11** shows the results of inhibition measurements of IL-1β-mediated IL-6 production with IL1R-FcV-RAcP-FcII heterodimer and IL-6 recovery from the culture medium where MRC5 cells were incubated for 24 hours with IL-1β at a final concentration of 50 pg/ml (IL-1 alone) or left untreated (Cells Ctrl) while culture medium not exposed to the cells was spiked with IL-6 at a final concentration of 30 pg/ml to estimate IL-6 recovery (RPH-10 + 30 pg/ml IL6); to ensure consistency between two cell culture plates used for this experiment, the effects of IL-1 on IL-6 production from both plates were compared (IL-1 alone and IL1 alone Plate 2, for plates 1 and 2 respectively); the effect of the highest concentration (20 µg/ml) of II,1R-FcV-RAcP-FcΠ heterodimer on IL-6 production was also tested (RPH-10 alone); IL1R-FcV-RAcP-FcII heterodimer dilution corresponding to a final concentration of 204.8 ng/ml was also included to demonstrate uniformity of the data between the two plates (RPH10 Plate 2); and
**Figure 12** shows the results of the titration curve measurements for L1R-FcV-RAcP-FcII heterodimer experiment where MRC5 cells were incubated with IL-1β or IL-1β pre-mixed with various concentrations of L1R-FcV-RAcP-FcII heterodimer; IL-6 production was measured by Quantakine ELISA and the data was analyzed using 4-parameter fit algorithm; coefficient C at the resulting equation is numerically equal to an IC50 value of 0.3 ng/ml or about 2.4 pM.

### DETAILED DESCRIPTION OF THE INVENTION

The teachings disclosed herein are based, in part, upon engineering of a heterodimeric protein assembly that is capable of binding to human IL-1 β and attenuating its function. The heterodimeric protein assembly of the present teachings comprises extracellular portions of IL1-R1 (GenBank: AAM88423.1) and of IL-1RAcP (GenBank: BAA25421.1), or functional fragments thereof. Each, the IL1-R1 portion and the IL-1RAcP portion, is fused to a distinct mutant of Fc portion of the human Ig Gamma-1 (GenBank: J00228.1). The two distinct Fc mutants in the heterodimeric protein assembly are engineered as to favor the heteromeric dimer formation between the two Fc mutants over any homomeric assembly. To enable recombinant production of the heterodimeric protein assembly of the present teachings, a DNA expression vector has been constructed for overproducing the heterodimeric protein assembly in a heterologous protein expression system, and mammalian cells have been prepared stably expressing the heterodimeric protein assembly to a high expression level. A protein purification procedure has been devised allowing obtaining a physiologically relevant substantially pure preparation of the heterodimeric protein assembly of the present teachings. Thus purified protein molecule demonstrates a high degree of specific activity in an *in vitro* Enzyme-Linked Immunosorbent Assay (ELISA) using human IL-1β (GenBank: AAH08678.1). Unexpectedly, the protein molecule exhibits an acceptable pharmacokinetics profile upon subcutaneous animal administration, while not resulting in any body weight loss or adverse clinical events.

The terms used in this specification generally have their ordinary meanings in the art, within the context of this invention and in the specific context where each term is used. Certain terms are discussed below or elsewhere in the specification, to provide additional guidance to the practitioner in describing the compositions and methods of the invention and how to make and use them. The scope or meaning of any use of a term will be apparent from the specific context in which the term is used. "About" and "approximately" shall generally mean an acceptable degree of error for the quantity measured given the nature or precision of the measurements. Typically, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 5- fold and more preferably within 2-fold of a given value. Numerical quantities given herein are approximate unless stated otherwise, meaning that the term "about" or "approximately" can be inferred when not expressly stated.

The methods disclosed herein may include steps of comparing sequences to each other, including wild-type sequence to one or more mutants (sequence variants). Such comparisons typically comprise alignments of polymer sequences, e.g., using sequence alignment programs and/or algorithms that are well known in the art (for example, BLAST, FASTA and MEGALIGN, to name a few). The skilled artisan can readily appreciate that, in such alignments, where a mutation contains a residue insertion or deletion, the sequence alignment will introduce a "gap" (typically represented by a dash, or "A") in the polymer sequence not containing the inserted or deleted residue.

The methods disclosed herein may include statistical calculations, e.g. determination of IC50 or EC50 values, etc.. The skilled artisan can readily appreciate that such can be performed using a variety of commercially available software, e.g. PRISM (GraphPad Software Inc, La Jolla, CA, USA) or similar.

"Homologous," in all its grammatical forms and spelling variations, refers to the relationship between two proteins that possess a "common evolutionary origin," including proteins from superfamilies in the same species of organism, as well as homologous proteins from different species of organism. Such proteins (and their encoding nucleic acids) have sequence homology, as reflected by their sequence similarity, whether in terms of percent identity or by the presence of specific residues or motifs and conserved positions. However, in common usage and in the instant application, the term "homologous," when modified with an adverb such as "highly," may refer to sequence similarity and may or may not relate to a common evolutionary origin.

The term "sequence similarity," in all its grammatical forms, refers to the degree of identity or correspondence between nucleic acid or amino acid sequences that may or may not share a common evolutionary origin.

The terms "protein" and "polypeptide" are used interchangeably. The polypeptides described herein may be comprised of more than one contiguous amino acid chain, thus forming dimers or other oligomeric formations. In general, the polypeptides described herein for use in mammals are expressed in mammalian cells that allow for proper post-translational modifications, such as CHO or HEK293 cell lines, although other mammalian expression cell lines are expected to be useful as well. It is therefore anticipated that the polypeptides described herein may be post-translationally modified without substantially effecting its biological function.

In certain aspects, functional variants of the heterodimeric protein assemblies described herein include fusion proteins having at least a biologically active portion of the human IL1-R1 or IL-1RAcP or a functional fragment thereof, and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (e.g., an Fc), maltose binding protein (MBP), or human serum albumin. A fusion domain may be selected so as to confer a desired property. For example, the IL1-R1 or IL-1RAcP polypeptide portions may be fused with a domain that stabilizes the IL1-R1 or IL-1RAcP polypeptides *in vivo* (a "stabilizer" domain), optionally via a suitable peptide linker. The term "stabilizing" means anything that increases the half life of a polypeptide in systemic circulation, regardless of whether this is because of decreased destruction, decreased clearance, or other pharmacokinetic effect. Fusions with the Fc portion of an immunoglobulin are known to confer desirable pharmacokinetic properties on certain proteins. Likewise, fusions to human serum albumin can confer desirable properties. Other types of fusion domains that may be selected include multimerizing (e.g., dimerizing, tetramerizing) domains and functional domains that confer an additional biological function, e.g. promoting accumulation at the targeted site of action *in vivo.*

In certain aspects, the heterodimeric protein assemblies described herein comprise an extracellular portion of IL1-R1, or a functional fragment thereof, fused with a IgG-Fc domain, and an extracellular portion IL-1RAcP, or a functional fragment thereof, fused with another IgG-Fc domain. The IgG-Fc domain and the another IgG-Fc domain are chosen as to favor a heterodimeric protein assembly over any homodimeric protein assembly. The extracellular portion of IL1-R1 may be fused with the IgG-Fc domain via a flexible linker, while IL-1RAcP, or a functional fragment thereof, may be fused with the another IgG-Fc domain via the flexible linker of the same amino acid sequence or via another flexible linker.

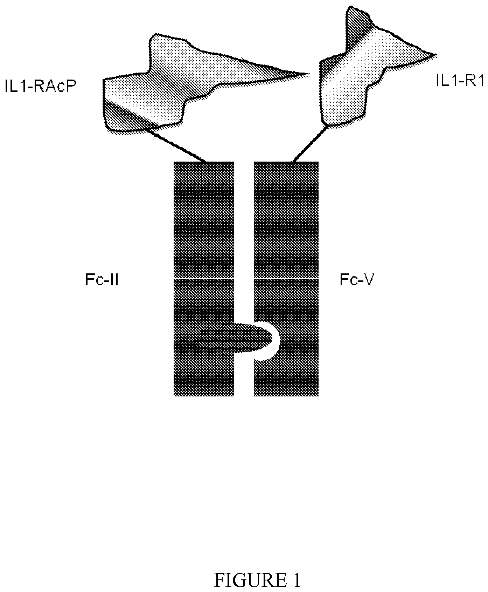

In an example embodiment, illustratively shown in **Figure 1****,** the extracellular portion of IL1-R1 fused with IgG-Fc domain (Fc-II) via a flexible linker may comprise the amino acid sequence of SEQ. ID NO. 1, while IL-1RAcP fused with another IgG-Fc domain (Fc-V) via a flexible linker may comprise the amino acid sequence of SEQ. ID NO. 2.
HIL1-R1-hIgG1-Fc polypeptide (SEQ ID NO. 1) 
LEADKCKERE EKIILVSSAN EIDVRPCPLN PNEHKGTITW YKDDSKTPVS TEQASRIHQH 60
KEKLWFVPAK VEDSGHYYCV VRNSSYCLRI KISAKFVENE PNLCYNAQAI FKQKLPVAGD 120
GGLVCPYMEF FKNENNELPK LQWYKDCKPL LLDNIHFSGV KDRLIVMNVA EKHRGNYTCH 180
ASYTYLGKQY PITRVIEFIT LEENKPTRPV IVSPANETME VDLGSQIQLI CNVTGQLSDI 240
AYWKWNGSVI DEDDPVLGED YYSVENPANK RRSTLITVLN ISEIESRFYK HPFTCFAKNT 300
HGIDAAYIQL IYPVTNGSGG GDKTHTCPPC PAPELLGGPS VFLFPPKPKD TLMISRTPEV 360
TCVVVDVSHE DPEVKFNWYV DGVEVHNAKT KPREEQYNST YRVVSVLTVL HQDWLNGKEY 420
KCKVSNKALP APIEKTISKA KGQPREPQVC TLPPSRDELT KNQVSLSCAV KGFYPSDIAV 480
EWESNGQPEN NYKTTPPVLD SDGSFKLVSK LTVDKSRWQQ GNVFSCSVMH EALHNHYTQK 540
SLSLSPGK 548
hIL-1RAcP-hIgG1-Fc polypeptide (SEQ ID NO. 2)

SERCDDWGLD TMRQIQVFED EPARIKCPLF EHFLKFNYST AHSAGLTLIW YWTRQDRDLE 60
EPINFRLPEN RISKEKDVLW FRPTLLNDTG NYTCMLRNTT YCSKVAFPLE VVQKDSCFNS 120

PMKLPVHKLY IEYGIQRITC PNVDGYFPSS VKPTITWYMG CYKIQNFNNV IPEGMNLSFL 180
IALISNNGNY TCVVTYPENG RTFHLTRTLT VKVVGSPKNA VPPVIHSPND HVVYEKEPGE 240
ELLIPCTVYF SFLMDSRNEV WWTIDGKKPD DITIDVTINE SISHSRTEDE TRTQILSIKK 300
VTSEDLKRSY VCHARSAKGE VAKAAKVKQK VPAPRYTVGS GGGDKTHTCP PCPAPELLGG 360
PSVFLFPPKP KDTLMISRTP EVTCVVVDVS HEDPEVKFNW YVDGVEVHNA KTKPREEQYN 420
STYRVVSVLT VLHQDWLNGK EYKCKVSNKA LPAPIEKTIS KAKGQPREPQ VYTLPPCRDE 480
LTKNQVSLWC LVKGFYPSDI AVEWESNGQP ENNYKTTPPV LDSDGSFFLY SALTVDKSRW 540
QQGNVFSCSV MHEALHNHYT QKSLSLSPGK 570

In certain aspects, the present teachings provides for a recombinant DNA molecule having an open reading frame coding for a polypeptide comprising the leading 333 amino acids of the human IL1-R1 fused with IgG-Fc domain (Fc-II) via a flexible linker, and for another recombinant DNA molecule having an open reading frame coding for another polypeptide comprising the leading 358 amino acids of the human IL-1RAcP fused with another IgG-Fc domain (Fc-V) via a flexible linker.

In an example embodiment, the polypeptide comprising the leading 333 amino acids of the human IL1-R1 fused with IgG-Fc domain (Fc-II) via a flexible linker comprises the amino acid sequence of SEQ. ID NO. 3. The corresponding to it DNA molecule may comprise the nucleotide sequence of SEQ ID NO. 4. The another polypeptide comprises the leading 358 amino acids of the human IL-1RAcP fused with another IgG-Fc domain (Fc-V) via a flexible linker may comprise the amino acid sequence of SEQ. ID NO. 5. The corresponding to it DNA molecule may comprise the nucleotide sequence of SEQ ID NO. 6.
HIL1-R1-hIgG1-Fc polypeptide (SEQ ID NO. 3) 
MKVLLRLICF IALLISSLEA DKCKEREEKI ILVSSANEID VRPCPLNPNE HKGTITWYKD 60
DSKTPVSTEQ ASRIHQHKEK LWFVPAKVED SGHYYCVVRN SSYCLRIKIS AKFVENEPNL 120
CYNAQAIFKQ KLPVAGDGGL VCPYMEFFKN ENNELPKLQW YKDCKPLLLD NIHFSGVKDR 180
LIVMNVAEKH RGNYTCHASY TYLGKQYPIT RVIEFITLEE NKPTRPVIVS PANETMEVDL 240
GSQIQLICNV TGQLSDIAYW KWNGSVIDED DPVLGEDYYS VENPANKRRS TLITVLNISE 300
IESRFYKHPF TCFAKNTHGI DAAYIQLIYP VTNGSGGGDK THTCPPCPAP ELLGGPSVFL 360
FPPKPKDTLM ISRTPEVTCV VVDVSHEDPE VKFNWYVDGV EVHNAKTKPR EEQYNSTYRV 420
VSVLTVLHQD WLNGKEYKCK VSNKALPAPI EKTISKAKGQ PREPQVCTLP PSRDELTKNQ 480
 
VSLSCAVKGF YPSDIAVEWE SNGQPENNYK TTPPVLDSDG SFKLVSKLTV DKSRWQQGNV 540

FSCSVMHEAL HNHYTQKSLS LSPGK 565
HIL1-R1-hIgG1-Fc DNA (SEQ ID NO. 4) 
ATGAAGGTCC TGCTCAGGCT GATCTGCTTC ATTGCCCTGC TCATCAGCAG CCTGGAAGCC 60
GACAAGTGCA AGGAGAGGGA GGAGAAGATC ATCCTCGTCA GCTCCGCCAA CGAGATTGAT 120
GTCAGGCCCT GCCCCCTCAA CCCCAATGAG CACAAGGGCA CAATCACCTG GTACAAGGAC 180
GACAGCAAGA CCCCTGTCTC CACCGAGCAG GCCAGCAGAA TCCACCAGCA CAAAGAGAAG 240
CTGTGGTTCG TGCCTGCCAA GGTGGAAGAC AGCGGCCACT ACTACTGTGT GGTGAGGAAC 300
AGCTCCTACT GCCTCAGGAT CAAGATCTCC GCCAAGTTCG TGGAGAACGA GCCCAACCTC 360
TGTTACAACG CTCAGGCTAT TTTCAAGCAA AAGCTCCCCG TGGCTGGAGA CGGAGGCCTG 420
GTCTGTCCCT ACATGGAGTT CTTCAAGAAT GAGAATAATG AGCTCCCCAA GCTCCAGTGG 480
TACAAGGACT GTAAGCCTCT GCTCCTGGAC AACATCCACT TCTCCGGCGT GAAGGACAGA 540
CTGATCGTCA TGAACGTGGC CGAGAAGCAC AGGGGAAACT ACACCTGTCA CGCCTCCTAC 600
ACCTACCTCG GCAAGCAATA TCCCATCACC AGGGTCATCG AGTTCATCAC CCTCGAAGAG 660
AACAAGCCCA CAAGGCCTGT CATCGTCAGC CCCGCCAATG AAACCATGGA GGTGGACCTC 720
GGCAGCCAGA TCCAGCTGAT CTGCAACGTG ACAGGCCAGC TCAGCGACAT TGCCTACTGG 780
AAGTGGAACG GCTCCGTGAT CGACGAAGAT GATCCCGTGC TGGGCGAGGA CTACTATAGC 840
GTGGAGAACC CCGCCAACAA AAGAAGGAGC ACCCTGATCA CCGTGCTGAA CATCAGCGAG 900
ATCGAGTCCA GATTCTATAA GCATCCTTTC ACCTGCTTTG CCAAGAACAC CCACGGCATC 960
GACGCCGCTT ACATCCAGCT GATCTATCCC GTGACCAACG GATCCGGTGG AGGTGACAAA 1020
ACTCACACAT GCCCACCGTG CCCAGCTCCG GAACTCCTGG GCGGACCGTC AGTCTTCCTC 1080
TTCCCCCCAA AACCCAAGGA CACCCTCATG ATCTCCCGGA CCCCTGAGGT CACATGCGTG 1140
GTGGTGGACG TGAGCCACGA AGACCCTGAG GTCAAGTTCA ACTGGTACGT GGACGGCGTG 1200
GAGGTGCATA ATGCCAAGAC AAAGCCGCGG GAGGAGCAGT ACAACAGCAC GTACCGTGTG 1260
GTCAGCGTCC TCACCGTCCT GCACCAGGAC TGGCTGAATG GCAAGGAGTA CAAGTGCAAG 1320
GTCTCCAACA AAGCCCTCCC AGCCCCCATC GAGAAAACCA TCTCCAAAGC CAAAGGGCAG 1380
CCCCGAGAAC CACAGGTGTG TACCCTGCCC CCATCCCGGG ATGAGCTGAC CAAGAACCAG 1440
GTCAGCCTGA GTTGCGCGGT CAAAGGCTTC TATCCCAGCG ACATCGCCGT GGAGTGGGAG 1500
AGCAATGGGC AGCCGGAGAA CAACTACAAG ACCACGCCTC CCGTGTTGGA CTCCGACGGC 1560
TCCTTCAAGC TCGTCAGCAA GCTCACCGTG GACAAGAGCA GGTGGCAGCA GGGGAACGTC 1620
TTCTCATGCT CCGTGATGCA TGAGGCTCTG CACAACCACT ACACGCAGAA GAGCCTCTCC 1680
 CTGTCTCCGG GTAAA 1695
hIL-1RAcP-hIgG1-Fc polypeptide (SEQ ID NO. 5) 
MTLLWCVVSL YFYGILQSDA SERCDDWGLD TMRQIQVFED EPARIKCPLF EHFLKFNYST 60
AHSAGLTLIW YWTRQDRDLE EPINFRLPEN RISKEKDVLW FRPTLLNDTG NYTCMLRNTT 120
YCSKVAFPLE VVQKDSCFNS PMKLPVHKLY IEYGIQRITC PNVDGYFPSS VKPTITWYMG 180
CYKIQNFNNV IPEGMNLSFL IALISNNGNY TCVVTYPENG RTFHLTRTLT VKVVGSPKNA 240
VPPVIHSPND HVVYEKEPGE ELLIPCTVYF SFLMDSRNEV WWTIDGKKPD DITIDVTINE 300
SISHSRTEDE TRTQILSIKK VTSEDLKRSY VCHARSAKGE VAKAAKVKQK VPAPRYTVGS 360
GGGDKTHTCP PCPAPELLGG PSVFLFPPKP KDTLMISRTP EVTCVVVDVS HEDPEVKFNW 420
YVDGVEVHNA KTKPREEQYN STYRVVSVLT VLHQDWLNGK EYKCKVSNKA LPAPIEKTIS 480
KAKGQPREPQ VYTLPPCRDE LTKNQVSLWC LVKGFYPSDI AVEWESNGQP ENNYKTTPPV 540
LDSDGSFFLY SALTVDKSRW QQGNVFSCSV MHEALHNHYT QKSLSLSPGK 590
hIL-1RAcP-hIgG1-Fc DNA (SEQ ID NO. 6)

ATGACTCTGC TGTGGTGCGT CGTGTCCCTC TACTTCTACG GCATCCTCCA GTCCGACGCC 60
AGCGAGAGGT GCGATGACTG GGGCCTGGAC ACCATGAGGC AGATCCAGGT GTTTGAGGAC 120
GAGCCTGCCA GGATTAAGTG CCCCCTCTTC GAGCACTTTC TGAAGTTCAA CTACAGCACC 180
GCTCACAGCG CTGGCCTGAC ACTGATCTGG TACTGGACAA GGCAGGACAG GGATCTCGAG 240
GAGCCCATCA ACTTCAGGCT GCCCGAAAAC AGAATCAGCA AGGAGAAGGA CGTGCTGTGG 300
TTCAGACCCA CCCTCCTCAA CGACACAGGC AACTACACCT GCATGCTCAG GAACACCACC 360
TACTGCAGCA AGGTGGCCTT CCCTCTCGAG GTGGTCCAGA AGGACAGCTG CTTCAACAGC 420
CCCATGAAGC TGCCCGTCCA TAAACTGTAC ATCGAGTACG GCATCCAGAG GATCACATGC 480
CCCAACGTGG ACGGCTACTT CCCCAGCTCC GTGAAGCCCA CCATCACATG GTACATGGGC 540
TGTTACAAAA TCCAGAACTT TAACAACGTC ATCCCCGAGG GCATGAATCT GTCCTTCCTG 600
ATCGCCCTGA TCAGCAACAA CGGCAATTAC ACCTGCGTCG TGACCTACCC CGAAAACGGC 660
AGGACCTTCC ACCTGACCAG GACCCTGACC GTGAAAGTCG TGGGAAGCCC CAAGAATGCC 720
GTGCCCCCCG TGATCCATTC CCCCAACGAC CACGTGGTGT ACGAGAAGGA GCCTGGAGAG 780
GAGCTGCTGA TCCCCTGCAC AGTGTACTTC TCCTTCCTGA TGGACTCCAG GAATGAAGTG 840
TGGTGGACCA TCGACGGCAA GAAGCCTGAC GACATCACCA TCGATGTGAC CATCAACGAG 900
AGCATCAGCC ACAGCAGGAC CGAGGACGAG ACCAGGACCC AGATCCTGAG CATCAAGAAA 960
GTCACCAGCG AGGACCTCAA GAGAAGCTAC GTCTGTCACG CCAGAAGCGC CAAAGGCGAG 1020

GTGGCCAAGG CTGCTAAGGT GAAACAGAAA GTGCCCGCTC CTAGGTACAC AGTCGGATCC 1080
GGTGGAGGTG ACAAAACTCA CACATGCCCA CCGTGCCCAG CTCCGGAACT CCTGGGCGGA 1140
CCGTCAGTCT TCCTCTTCCC CCCAAAACCC AAGGACACCC TCATGATCTC CCGGACCCCT 1200
GAGGTCACAT GCGTGGTGGT GGACGTGAGC CACGAAGACC CTGAGGTCAA GTTCAACTGG 1260
TACGTGGACG GCGTGGAGGT GCATAATGCC AAGACAAAGC CGCGGGAGGA GCAGTACAAC 1320
AGCACGTACC GTGTGGTCAG CGTCCTCACC GTCCTGCACC AGGACTGGCT GAATGGCAAG 1380
GAGTACAAGT GCAAGGTCTC CAACAAAGCC CTCCCAGCCC CCATCGAGAA AACCATCTCC 1440
AAAGCCAAAG GGCAGCCCCG AGAACCACAG GTGTACACCC TGCCCCCATG TCGGGATGAG 1500
CTGACCAAGA ACCAGGTCAG CCTGTGGTGC CTGGTCAAAG GCTTCTATCC CAGCGACATC 1560
GCCGTGGAGT GGGAGAGCAA TGGGCAGCCG GAGAACAACT ACAAGACCAC GCCTCCCGTG 1620
TTGGACTCCG ACGGCTCCTT CTTCCTCTAC AGCGCGCTCA CCGTGGACAA GAGCAGGTGG 1680
CAGCAGGGGA ACGTCTTCTC ATGCTCCGTG ATGCATGAGG CTCTGCACAA CCACTACACG 1740
CAGAAGAGCC TCTCCCTGTC TCCGGGTAAA 1770

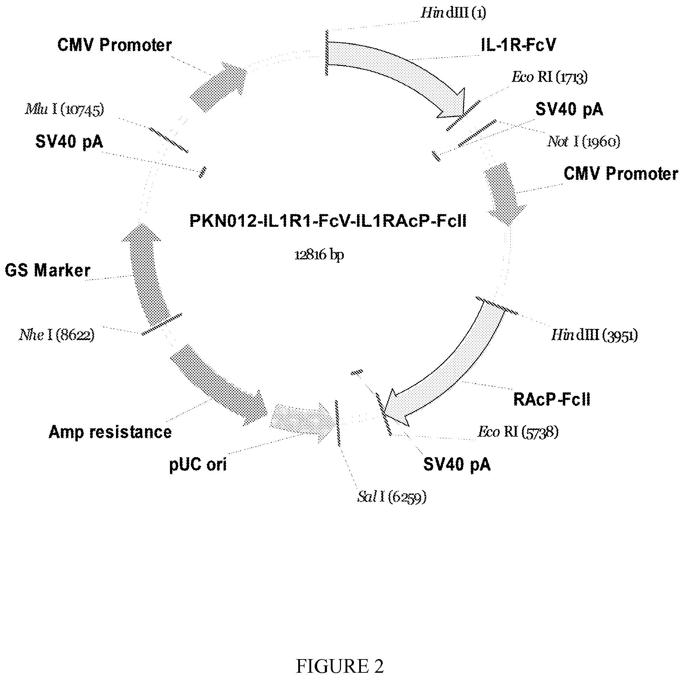

In certain aspects, the present disclosure provides for a recombinant mamalian expression plasmid for high expression of a polypeptide comprising the leading 333 amino acids of the human IL1-R1 fused with IgG-Fc domain (Fc-II) via a flexible linker, and for another recombinant DNA molecule having an open reading frame coding for another polypeptide comprising the leading 358 amino acids of the human IL-1RAcP fused with another IgG-Fc domain (Fc-V) via a flexible linker. This plasmid comprises two cytomegalovirus (CMV) promoters to drive transcription of the two genes coding for said polypeptide and said another polypeptide, each followed by a transcription termination sequence and a polyadenylation sequence. The plasmid also contains an origin of replication and a gene conferring ampicillin resistance, for supporting plasmid propagation and selection in bacteria. The plasmid further contains a gene for Glutamine synthetase, a selectable marker widely used for establishing stable CHOK1 and NSO cell lines. The plasmid of the present disclosure is illustratively shown in **Figure 2****.**

In an example embodiment, the mammalian expression plasmid of the present teachings comprises the nucleotide sequence of SEQ ID NO. 7.
HIL1-R1-hIgG1-Fc-II/ IL-1RAcP- hIgG1-Fc-V expression plasmid (SEQ ID NO. 7)

AGCTTGCCAC CATGAAGGTC CTGCTCAGGC TGATCTGCTT CATTGCCCTG CTCATCAGCA 60
GCCTGGAAGC CGACAAGTGC AAGGAGAGGG AGGAGAAGAT CATCCTCGTC AGCTCCGCCA 120

ACGAGATTGA TGTCAGGCCC TGCCCCCTCA ACCCCAATGA GCACAAGGGC ACAATCACCT 180
GGTACAAGGA CGACAGCAAG ACCCCTGTCT CCACCGAGCA GGCCAGCAGA ATCCACCAGC 240
ACAAAGAGAA GCTGTGGTTC GTGCCTGCCA AGGTGGAAGA CAGCGGCCAC TACTACTGTG 300
TGGTGAGGAA CAGCTCCTAC TGCCTCAGGA TCAAGATCTC CGCCAAGTTC GTGGAGAACG 360
AGCCCAACCT CTGTTACAAC GCTCAGGCTA TTTTCAAGCA AAAGCTCCCC GTGGCTGGAG 420
ACGGAGGCCT GGTCTGTCCC TACATGGAGT TCTTCAAGAA TGAGAATAAT GAGCTCCCCA 480
AGCTCCAGTG GTACAAGGAC TGTAAGCCTC TGCTCCTGGA CAACATCCAC TTCTCCGGCG 540
TGAAGGACAG ACTGATCGTC ATGAACGTGG CCGAGAAGCA CAGGGGAAAC TACACCTGTC 600
ACGCCTCCTA CACCTACCTC GGCAAGCAAT ATCCCATCAC CAGGGTCATC GAGTTCATCA 660
CCCTCGAAGA GAACAAGCCC ACAAGGCCTG TCATCGTCAG CCCCGCCAAT GAAACCATGG 720
AGGTGGACCT CGGCAGCCAG ATCCAGCTGA TCTGCAACGT GACAGGCCAG CTCAGCGACA 780
TTGCCTACTG GAAGTGGAAC GGCTCCGTGA TCGACGAAGA TGATCCCGTG CTGGGCGAGG 840
ACTACTATAG CGTGGAGAAC CCCGCCAACA AAAGAAGGAG CACCCTGATC ACCGTGCTGA 900
ACATCAGCGA GATCGAGTCC AGATTCTATA AGCATCCTTT CACCTGCTTT GCCAAGAACA 960
CCCACGGCAT CGACGCCGCT TACATCCAGC TGATCTATCC CGTGACCAAC GGATCCGGTG 1020
GAGGTGACAA AACTCACACA TGCCCACCGT GCCCAGCTCC GGAACTCCTG GGCGGACCGT 1080
CAGTCTTCCT CTTCCCCCCA AAACCCAAGG ACACCCTCAT GATCTCCCGG ACCCCTGAGG 1140
TCACATGCGT GGTGGTGGAC GTGAGCCACG AAGACCCTGA GGTCAAGTTC AACTGGTACG 1200
TGGACGGCGT GGAGGTGCAT AATGCCAAGA CAAAGCCGCG GGAGGAGCAG TACAACAGCA 1260
CGTACCGTGT GGTCAGCGTC CTCACCGTCC TGCACCAGGA CTGGCTGAAT GGCAAGGAGT 1320
ACAAGTGCAA GGTCTCCAAC AAAGCCCTCC CAGCCCCCAT CGAGAAAACC ATCTCCAAAG 1380
CCAAAGGGCA GCCCCGAGAA CCACAGGTGT GTACCCTGCC CCCATCCCGG GATGAGCTGA 1440
CCAAGAACCA GGTCAGCCTG AGTTGCGCGG TCAAAGGCTT CTATCCCAGC GACATCGCCG 1500
TGGAGTGGGA GAGCAATGGG CAGCCGGAGA ACAACTACAA GACCACGCCT CCCGTGTTGG 1560
ACTCCGACGG CTCCTTCAAG CTCGTCAGCA AGCTCACCGT GGACAAGAGC AGGTGGCAGC 1620
AGGGGAACGT CTTCTCATGC TCCGTGATGC ATGAGGCTCT GCACAACCAC TACACGCAGA 1680
AGAGCCTCTC CCTGTCTCCG GGTAAATAAT AGAATTCATT GATCATAATC AGCCATACCA 1740
CATTTGTAGA GGTTTTACTT GCTTTAAAAA ACCTCCCACA CCTCCCCCTG AACCTGAAAC 1800
ATAAAATGAA TGCAATTGTT GTTGTTAACT TGTTTATTGC AGCTTATAAT GGTTACAAAT 1860
AAAGCAATAG CATCACAAAT TTCACAAATA AAGCATTTTT TTCACTGCAT TCTAGTTGTG 1920
GTTTGTCCAA ACTCATCAAT GTATCTTATC ATGTCTGGCG GCCGCCGATA TTTGAAAATA 1980
TGGCATATTG AAAATGTCGC CGATGTGAGT TTCTGTGTAA CTGATATCGC CATTTTTCCA 2040
AAAGTGATTT TTGGGCATAC GCGATATCTG GCGATAGCGC TTATATCGTT TACGGGGGAT 2100
GGCGATAGAC GACTTTGGTG ACTTGGGCGA TTCTGTGTGT CGCAAATATC GCAGTTTCGA 2160
TATAGGTGAC AGACGATATG AGGCTATATC GCCGATAGAG GCGACATCAA GCTGGCACAT 2220

GGCCAATGCA TATCGATCTA TACATTGAAT CAATATTGGC CATTAGCCAT ATTATTCATT 2280
GGTTATATAG CATAAATCAA TATTGGCTAT TGGCCATTGC ATACGTTGTA TCCATATCAT 2340
AATATGTACA TTTATATTGG CTCATGTCCA ACATTACCGC CATGTTGACA TTGATTATTG 2400
ACTAGTTATT AATAGTAATC AATTACGGGG TCATTAGTTC ATAGCCCATA TATGGAGTTC 2460
CGCGTTACAT AACTTACGGT AAATGGCCCG CCTGGCTGAC CGCCCAACGA CCCCCGCCCA 2520
TTGACGTCAA TAATGACGTA TGTTCCCATA GTAACGCCAA TAGGGACTTT CCATTGACGT 2580
CAATGGGTGG AGTATTTACG GTAAACTGCC CACTTGGCAG TACATCAAGT GTATCATATG 2640
CCAAGTACGC CCCCTATTGA CGTCAATGAC GGTAAATGGC CCGCCTGGCA TTATGCCCAG 2700
TACATGACCT TATGGGACTT TCCTACTTGG CAGTACATCT ACGTATTAGT CATCGCTATT 2760
ACCATGGTGA TGCGGTTTTG GCAGTACATC AATGGGCGTG GATAGCGGTT TGACTCACGG 2820
GGATTTCCAA GTCTCCACCC CATTGACGTC AATGGGAGTT TGTTTTGGCA CCAAAATCAA 2880
CGGGACTTTC CAAAATGTCG TAACAACTCC GCCCCATTGA CGCAAATGGG CGGTAGGCGT 2940
GTACGGTGGG AGGTCTATAT AAGCAGAGCT CGTTTAGTGA ACCGTCAGAT CGCCTGGAGA 3000
CGCCATCCAC GCTGTTTTGA CCTCCATAGA AGACACCGGG ACCGATCCAG CCTCCGCGGC 3060
CGGGAACGGT GCATTGGAAC GCGGATTCCC CGTGCCAAGA GTGACGTAAG TACCGCCTAT 3120
AGAGTCTATA GGCCCACCCC CTTGGCTTCT TATGCATGCT ATACTGTTTT TGGCTTGGGG 3180
TCTATACACC CCCGCTTCCT CATGTTATAG GTGATGGTAT AGCTTAGCCT ATAGGTGTGG 3240
GTTATTGACC ATTATTGACC ACTCCCCTAT TGGTGACGAT ACTTTCCATT ACTAATCCAT 3300
AACATGGCTC TTTGCCACAA CTCTCTTTAT TGGCTATATG CCAATACACT GTCCTTCAGA 3360
GACTGACACG GACTCTGTAT TTTTACAGGA TGGGGTCTCA TTTATTATTT ACAAATTCAC 3420
ATATACAACA CCACCGTCCC CAGTGCCCGC AGTTTTTATT AAACATAACG TGGGATCTCC 3480
ACGCGAATCT CGGGTACGTG TTCCGGACAT GGGCTCTTCT CCGGTAGCGG CGGAGCTTCT 3540
ACATCCGAGC CCTGCTCCCA TGCCTCCAGC GACTCATGGT CGCTCGGCAG CTCCTTGCTC 3600
CTAACAGTGG AGGCCAGACT TAGGCACAGC ACGATGCCCA CCACCACCAG TGTGCCGCAC 3660
AAGGCCGTGG CGGTAGGGTA TGTGTCTGAA AATGAGCTCG GGGAGCGGGC TTGCACCGCT 3720
GACGCATTTG GAAGACTTAA GGCAGCGGCA GAAGAAGATG CAGGCAGCTG AGTTGTTGTG 3780
TTCTGATAAG AGTCAGAGGT AACTCCCGTT GCGGTGCTGT TAACGGTGGA GGGCAGTGTA 3840
GTCTGAGCAG TACTCGTTGC TGCCGCGCGC GCCACCAGAC ATAATAGCTG ACAGACTAAC 3900
AGACTGTTCC TTTCCATGGG TCTTTTCTGC AGTCACCGTC CTTGACACGA AGCTTGCCAC 3960
CATGACTCTG CTGTGGTGCG TCGTGTCCCT CTACTTCTAC GGCATCCTCC AGTCCGACGC 4020
CAGCGAGAGG TGCGATGACT GGGGCCTGGA CACCATGAGG CAGATCCAGG TGTTTGAGGA 4080
CGAGCCTGCC AGGATTAAGT GCCCCCTCTT CGAGCACTTT CTGAAGTTCA ACTACAGCAC 4140
CGCTCACAGC GCTGGCCTGA CACTGATCTG GTACTGGACA AGGCAGGACA GGGATCTCGA 4200
GGAGCCCATC AACTTCAGGC TGCCCGAAAA CAGAATCAGC AAGGAGAAGG ACGTGCTGTG 4260
GTTCAGACCC ACCCTCCTCA ACGACACAGG CAACTACACC TGCATGCTCA GGAACACCAC 4320

CTACTGCAGC AAGGTGGCCT TCCCTCTCGA GGTGGTCCAG AAGGACAGCT GCTTCAACAG 4380
CCCCATGAAG CTGCCCGTCC ATAAACTGTA CATCGAGTAC GGCATCCAGA GGATCACATG 4440
CCCCAACGTG GACGGCTACT TCCCCAGCTC CGTGAAGCCC ACCATCACAT GGTACATGGG 4500
CTGTTACAAA ATCCAGAACT TTAACAACGT CATCCCCGAG GGCATGAATC TGTCCTTCCT 4560
GATCGCCCTG ATCAGCAACA ACGGCAATTA CACCTGCGTC GTGACCTACC CCGAAAACGG 4620
CAGGACCTTC CACCTGACCA GGACCCTGAC CGTGAAAGTC GTGGGAAGCC CCAAGAATGC 4680
CGTGCCCCCC GTGATCCATT CCCCCAACGA CCACGTGGTG TACGAGAAGG AGCCTGGAGA 4740
GGAGCTGCTG ATCCCCTGCA CAGTGTACTT CTCCTTCCTG ATGGACTCCA GGAATGAAGT 4800
GTGGTGGACC ATCGACGGCA AGAAGCCTGA CGACATCACC ATCGATGTGA CCATCAACGA 4860
GAGCATCAGC CACAGCAGGA CCGAGGACGA GACCAGGACC CAGATCCTGA GCATCAAGAA 4920
AGTCACCAGC GAGGACCTCA AGAGAAGCTA CGTCTGTCAC GCCAGAAGCG CCAAAGGCGA 4980
GGTGGCCAAG GCTGCTAAGG TGAAACAGAA AGTGCCCGCT CCTAGGTACA CAGTCGGATC 5040
CGGTGGAGGT GACAAAACTC ACACATGCCC ACCGTGCCCA GCTCCGGAAC TCCTGGGCGG 5100
ACCGTCAGTC TTCCTCTTCC CCCCAAAACC CAAGGACACC CTCATGATCT CCCGGACCCC 5160
TGAGGTCACA TGCGTGGTGG TGGACGTGAG CCACGAAGAC CCTGAGGTCA AGTTCAACTG 5220
GTACGTGGAC GGCGTGGAGG TGCATAATGC CAAGACAAAG CCGCGGGAGG AGCAGTACAA 5280
CAGCACGTAC CGTGTGGTCA GCGTCCTCAC CGTCCTGCAC CAGGACTGGC TGAATGGCAA 5340
GGAGTACAAG TGCAAGGTCT CCAACAAAGC CCTCCCAGCC CCCATCGAGA AAACCATCTC 5400
CAAAGCCAAA GGGCAGCCCC GAGAACCACA GGTGTACACC CTGCCCCCAT GTCGGGATGA 5460
GCTGACCAAG AACCAGGTCA GCCTGTGGTG CCTGGTCAAA GGCTTCTATC CCAGCGACAT 5520
CGCCGTGGAG TGGGAGAGCA ATGGGCAGCC GGAGAACAAC TACAAGACCA CGCCTCCCGT 5580
GTTGGACTCC GACGGCTCCT TCTTCCTCTA CAGCGCGCTC ACCGTGGACA AGAGCAGGTG 5640
GCAGCAGGGG AACGTCTTCT CATGCTCCGT GATGCATGAG GCTCTGCACA ACCACTACAC 5700
GCAGAAGAGC CTCTCCCTGT CTCCGGGTAA ATAATAGAAT TCATTGATCA TAATCAGCCA 5760
TACCACATTT GTAGAGGTTT TACTTGCTTT AAAAAACCTC CCACACCTCC CCCTGAACCT 5820
GAAACATAAA ATGAATGCAA TTGTTGTTGT TAACTTGTTT ATTGCAGCTT ATAATGGTTA 5880
CAAATAAAGC AATAGCATCA CAAATTTCAC AAATAAAGCA TTTTTTTCAC TGCATTCTAG 5940
TTGTGGTTTG TCCAAACTCA TCAATGTATC TTATCATGTC TGGATCCTCT ACGCCGGACG 6000
CATCGTGGCC GGCATCACCG GCGCCACAGG TGCGGTTGCT GGCGCCTATA TCGCCGACAT 6060
CACCGATGGG GAAGATCGGG CTCGCCACTT CGGGCTCATG AGCGCTTGTT TCGGCGTGGG 6120
TATGGTGGCA GGCCCCGTGG CCGGGGGACT GTTGGGCGCC ATCTCCTTGC ATGCACCATT 6180
CCTTGCGGCG GCGGTGCTCA ACGGCCTCAA CCTACTACTG GGCTGCTTCC TAATGCAGGA 6240
GTCGCATAAG GGAGAGCGTC GACCTCGGGC CGCGTTGCTG GCGTTTTTCC ATAGGCTCCG 6300
CCCCCCTGAC GAGCATCACA AAAATCGACG CTCAAGTCAG AGGTGGCGAA ACCCGACAGG 6360
ACTATAAAGA TACCAGGCGT TTCCCCCTGG AAGCTCCCTC GTGCGCTCTC CTGTTCCGAC 6420

CCTGCCGCTT ACCGGATACC TGTCCGCCTT TCTCCCTTCG GGAAGCGTGG CGCTTTCTCA 6480
TAGCTCACGC TGTAGGTATC TCAGTTCGGT GTAGGTCGTT CGCTCCAAGC TGGGCTGTGT 6540
GCACGAACCC CCCGTTCAGC CCGACCGCTG CGCCTTATCC GGTAACTATC GTCTTGAGTC 6600
CAACCCGGTA AGACACGACT TATCGCCACT GGCAGCAGCC ACTGGTAACA GGATTAGCAG 6660
AGCGAGGTAT GTAGGCGGTG CTACAGAGTT CTTGAAGTGG TGGCCTAACT ACGGCTACAC 6720
TAGAAGAACA GTATTTGGTA TCTGCGCTCT GCTGAAGCCA GTTACCTTCG GAAAAAGAGT 6780
TGGTAGCTCT TGATCCGGCA AACAAACCAC CGCTGGTAGC GGTGGTTTTT TTGTTTGCAA 6840
GCAGCAGATT ACGCGCAGAA AAAAAGGATC TCAAGAAGAT CCTTTGATCT TTTCTACGGG 6900
GTCTGACGCT CAGTGGAACG AAAACTCACG TTAAGGGATT TTGGTCATGA GATTATCAAA 6960
AAGGATCTTC ACCTAGATCC TTTTAAATTA AAAATGAAGT TTTAAATCAA TCTAAAGTAT 7020
ATATGAGTAA ACTTGGTCTG ACAGTTACCA ATGCTTAATC AGTGAGGCAC CTATCTCAGC 7080
GATCTGTCTA TTTCGTTCAT CCATAGTTGC CTGACTCCCC GTCGTGTAGA TAACTACGAT 7140
ACGGGAGGGC TTACCATCTG GCCCCAGTGC TGCAATGATA CCGCGAGACC CACGCTCACC 7200
GGCTCCAGAT TTATCAGCAA TAAACCAGCC AGCCGGAAGG GCCGAGCGCA GAAGTGGTCC 7260
TGCAACTTTA TCCGCCTCCA TCCAGTCTAT TAATTGTTGC CGGGAAGCTA GAGTAAGTAG 7320
TTCGCCAGTT AATAGTTTGC GCAACGTTGT TGCCATTGCT ACAGGCATCG TGGTGTCACG 7380
CTCGTCGTTT GGTATGGCTT CATTCAGCTC CGGTTCCCAA CGATCAAGGC GAGTTACATG 7440
ATCCCCCATG TTGTGCAAAA AAGCGGTTAG CTCCTTCGGT CCTCCGATCG TTGTCAGAAG 7500
TAAGTTGGCC GCAGTGTTAT CACTCATGGT TATGGCAGCA CTGCATAATT CTCTTACTGT 7560
CATGCCATCC GTAAGATGCT TTTCTGTGAC TGGTGAGTAC TCAACCAAGT CATTCTGAGA 7620
ATAGTGTATG CGGCGACCGA GTTGCTCTTG CCCGGCGTCA ATACGGGATA ATACCGCGCC 7680
ACATAGCAGA ACTTTAAAAG TGCTCATCAT TGGAAAACGT TCTTCGGGGC GAAAACTCTC 7740
AAGGATCTTA CCGCTGTTGA GATCCAGTTC GATGTAACCC ACTCGTGCAC CCAACTGATC 7800
TTCAGCATCT TTTACTTTCA CCAGCGTTTC TGGGTGAGCA AAAACAGGAA GGCAAAATGC 7860
CGCAAAAAAG GGAATAAGGG CGACACGGAA ATGTTGAATA CTCATACTCT TCCTTTTTCA 7920
ATATTATTGA AGCATTTATC AGGGTTATTG TCTCATGAGC GGATACATAT TTGAATGTAT 7980
TTAGAAAAAT AAACAAATAG GGGTTCCGCG CACATTTCCC CGAAAAGTGC CACCTGACGT 8040
CTAAGAAACC ATTATTATCA TGACATTAAC CTATAAAAAT AGGCGTATCA CGAGGCCCTG 8100
ATGGCTCTTT GCGGCACCCA TCGTTCGTAA TGTTCCGTGG CACCGAGGAC AACCCTCAAG 8160
AGAAAATGTA ATCACACTGG CTCACCTTCG GGTGGGCCTT TCTGCGTTTA TAAGGAGACA 8220
CTTTATGTTT AAGAAGGTTG GTAAATTCCT TGCGGCTTTG GCAGCCAAGC TAGATCCGGC 8280
TGTGGAATGT GTGTCAGTTA GGGTGTGGAA AGTCCCCAGG CTCCCCAGCA GGCAGAAGTA 8340
TGCAAAGCAT GCATCTCAAT TAGTCAGCAA CCAGGTGTGG AAAGTCCCCA GGCTCCCCAG 8400
CAGGCAGAAG TATGCAAAGC ATGCATCTCA ATTAGTCAGC AACCATAGTC CCGCCCCTAA 8460
CTCCGCCCAT CCCGCCCCTA ACTCCGCCCA GTTCCGCCCA TTCTCCGCCC CATGGCTGAC 8520

TAATTTTTTT TATTTATGCA GAGGCCGAGG CCGCCTCGGC CTCTGAGCTA TTCCAGAAGT 8580
AGTGAGGAGG CTTTTTTGGA GGCCTAGGCT TTTGCAAAAA GCTAGCTTGG GGCCACCGCT 8640
CAGAGCACCT TCCACCATGG CCACCTCAGC AAGTTCCCAC TTGAACAAAA ACATCAAGCA 8700
AATGTACTTG TGCCTGCCCC AGGGTGAGAA AGTCCAAGCC ATGTATATCT GGGTTGATGG 8760
TACTGGAGAA GGACTGCGCT GCAAAACCCG CACCCTGGAC TGTGAGCCCA AGTGTGTAGA 8820
AGAGTTACCT GAGTGGAATT TTGATGGCTC TAGTACCTTT CAGTCTGAGG GCTCCAACAG 8880
TGACATGTAT CTCAGCCCTG TTGCCATGTT TCGGGACCCC TTCCGCAGAG ATCCCAACAA 8940
GCTGGTGTTC TGTGAAGTTT TCAAGTACAA CCGGAAGCCT GCAGAGACCA ATTTAAGGCA 9000
CTCGTGTAAA CGGATAATGG ACATGGTGAG CAACCAGCAC CCCTGGTTTG GAATGGAACA 9060
GGAGTATACT CTGATGGGAA CAGATGGGCA CCCTTTTGGT TGGCCTTCCA ATGGCTTTCC 9120
TGGGCCCCAA GGTCCGTATT ACTGTGGTGT GGGCGCAGAC AAAGCCTATG GCAGGGATAT 9180
CGTGGAGGCT CACTACCGCG CCTGCTTGTA TGCTGGGGTC AAGATTACAG GAACAAATGC 9240
TGAGGTCATG CCTGCCCAGT GGGAACTCCA AATAGGACCC TGTGAAGGAA TCCGCATGGG 9300
AGATCATCTC TGGGTGGCCC GTTTCATCTT GCATCGAGTA TGTGAAGACT TTGGGGTAAT 9360
AGCAACCTTT GACCCCAAGC CCATTCCTGG GAACTGGAAT GGTGCAGGCT GCCATACCAA 9420
CTTTAGCACC AAGGCCATGC GGGAGGAGAA TGGTCTGAAG CACATCGAGG AGGCCATCGA 9480
GAAACTAAGC AAGCGGCACC GGTACCACAT TCGAGCCTAC GATCCCAAGG GGGGCCTGGA 9540
CAATGCCCGT GGTCTGACTG GGTTCCACGA AACGTCCAAC ATCAACGACT TTTCTGCTGG 9600
TGTCGCCAAT CGCAGTGCCA GCATCCGCAT TCCCCGGACT GTCGGCCAGG AGAAGAAAGG 9660
TTACTTTGAA GACCGCGGCC CCTCTGCCAA TTGTGACCCC TTTGCAGTGA CAGAAGCCAT 9720
CGTCCGCACA TGCCTTCTCA ATGAGACTGG CGACGAGCCC TTCCAATACA AAAACTAATT 9780
AGACTTTGAG TGATCTTGAG CCTTTCCTAG TTCATCCCAC CCCGCCCCAG AGAGATCTTT 9840
GTGAAGGAAC CTTACTTCTG TGGTGTGACA TAATTGGACA AACTACCTAC AGAGATTTAA 9900
AGCTCTAAGG TAAATATAAA ATTTTTAAGT GTATAATGTG TTAAACTACT GATTCTAATT 9960
GTTTGTGTAT TTTAGATTCC AACCTATGGA ACTGATGAAT GGGAGCAGTG GTGGAATGCC 10020
TTTAATGAGG AAAACCTGTT TTGCTCAGAA GAAATGCCAT CTAGTGATGA TGAGGCTACT 10080
GCTGACTCTC AACATTCTAC TCCTCCAAAA AAGAAGAGAA AGGTAGAAGA CCCCAAGGAC 10140
TTTCCTTCAG AATTGCTAAG TTTTTTGAGT CATGCTGTGT TTAGTAATAG AACTCTTGCT 10200
TGCTTTGCTA TTTACACCAC AAAGGAAAAA GCTGCACTGC TATACAAGAA AATTATGGAA 10260
AAATATTCTG TAACCTTTAT AAGTAGGCAT AACAGTTATA ATCATAACAT ACTGTTTTTT 10320
CTTACTCCAC ACAGGCATAG AGTGTCTGCT ATTAATAACT ATGCTCAAAA ATTGTGTACC 10380
TTTAGCTTTT TAATTTGTAA AGGGGTTAAT AAGGAATATT TGATGTATAG TGCCTTGACT 10440
AGAGATCATA ATCAGCCATA CCACATTTGT AGAGGTTTTA CTTGCTTTAA AAAACCTCCC 10500
ACACCTCCCC CTGAACCTGA AACATAAAAT GAATGCAATT GTTGTTGTTA ACTTGTTTAT 10560
TGCAGCTTAT AATGGTTACA AATAAAGCAA TAGCATCACA AATTTCACAA ATAAAGCATT 10620

TTTTTCACTG CATTCTAGTT GTGGTTTGTC CAAACTCATC AATGTATCTT ATCATGTCTG 10680
GATCTAGCTT CGTGTCAAGG ACGGTGACTG CAGTGAATAA TAAAATGTGT GTTTGTCCGA 10740
AATACGCGTT TTGAGATTTC TGTCGCCGAC TAAATTCATG TCGCGCGATA GTGGTGTTTA 10800
TCGCCGATAG AGATGGCGAT ATTGGAAAAA TCGATATTTG AAAATATGGC ATATTGAAAA 10860
TGTCGCCGAT GTGAGTTTCT GTGTAACTGA TATCGCCATT TTTCCAAAAG TGATTTTTGG 10920
GCATACGCGA TATCTGGCGA TAGCGCTTAT ATCGTTTACG GGGGATGGCG ATAGACGACT 10980
TTGGTGACTT GGGCGATTCT GTGTGTCGCA AATATCGCAG TTTCGATATA GGTGACAGAC 11040
GATATGAGGC TATATCGCCG ATAGAGGCGA CATCAAGCTG GCACATGGCC AATGCATATC 11100
GATCTATACA TTGAATCAAT ATTGGCCATT AGCCATATTA TTCATTGGTT ATATAGCATA 11160
AATCAATATT GGCTATTGGC CATTGCATAC GTTGTATCCA TATCATAATA TGTACATTTA 11220
TATTGGCTCA TGTCCAACAT TACCGCCATG TTGACATTGA TTATTGACTA GTTATTAATA 11280
GTAATCAATT ACGGGGTCAT TAGTTCATAG CCCATATATG GAGTTCCGCG TTACATAACT 11340
TACGGTAAAT GGCCCGCCTG GCTGACCGCC CAACGACCCC CGCCCATTGA CGTCAATAAT 11400
GACGTATGTT CCCATAGTAA CGCCAATAGG GACTTTCCAT TGACGTCAAT GGGTGGAGTA 11460
TTTACGGTAA ACTGCCCACT TGGCAGTACA TCAAGTGTAT CATATGCCAA GTACGCCCCC 11520
TATTGACGTC AATGACGGTA AATGGCCCGC CTGGCATTAT GCCCAGTACA TGACCTTATG 11580
GGACTTTCCT ACTTGGCAGT ACATCTACGT ATTAGTCATC GCTATTACCA TGGTGATGCG 11640
GTTTTGGCAG TACATCAATG GGCGTGGATA GCGGTTTGAC TCACGGGGAT TTCCAAGTCT 11700
CCACCCCATT GACGTCAATG GGAGTTTGTT TTGGCACCAA AATCAACGGG ACTTTCCAAA 11760
ATGTCGTAAC AACTCCGCCC CATTGACGCA AATGGGCGGT AGGCGTGTAC GGTGGGAGGT 11820
CTATATAAGC AGAGCTCGTT TAGTGAACCG TCAGATCGCC TGGAGACGCC ATCCACGCTG 11880
TTTTGACCTC CATAGAAGAC ACCGGGACCG ATCCAGCCTC CGCGGCCGGG AACGGTGCAT 11940
TGGAACGCGG ATTCCCCGTG CCAAGAGTGA CGTAAGTACC GCCTATAGAG TCTATAGGCC 12000
CACCCCCTTG GCTTCTTATG CATGCTATAC TGTTTTTGGC TTGGGGTCTA TACACCCCCG 12060
CTTCCTCATG TTATAGGTGA TGGTATAGCT TAGCCTATAG GTGTGGGTTA TTGACCATTA 12120
TTGACCACTC CCCTATTGGT GACGATACTT TCCATTACTA ATCCATAACA TGGCTCTTTG 12180
CCACAACTCT CTTTATTGGC TATATGCCAA TACACTGTCC TTCAGAGACT GACACGGACT 12240
CTGTATTTTT ACAGGATGGG GTCTCATTTA TTATTTACAA ATTCACATAT ACAACACCAC 12300
CGTCCCCAGT GCCCGCAGTT TTTATTAAAC ATAACGTGGG ATCTCCACGC GAATCTCGGG 12360
TACGTGTTCC GGACATGGGC TCTTCTCCGG TAGCGGCGGA GCTTCTACAT CCGAGCCCTG 12420
CTCCCATGCC TCCAGCGACT CATGGTCGCT CGGCAGCTCC TTGCTCCTAA CAGTGGAGGC 12480
CAGACTTAGG CACAGCACGA TGCCCACCAC CACCAGTGTG CCGCACAAGG CCGTGGCGGT 12540
AGGGTATGTG TCTGAAAATG AGCTCGGGGA GCGGGCTTGC ACCGCTGACG CATTTGGAAG 12600
ACTTAAGGCA GCGGCAGAAG AAGATGCAGG CAGCTGAGTT GTTGTGTTCT GATAAGAGTC 12660
AGAGGTAACT CCCGTTGCGG TGCTGTTAAC GGTGGAGGGC AGTGTAGTCT GAGCAGTACT 12720

CGTTGCTGCC GCGCGCGCCA CCAGACATAA TAGCTGACAG ACTAACAGAC TGTTCCTTTC 12780
CATGGGTCTT TTCTGCAGTC ACCGTCCTTG ACACGA 12816

In certain aspects, the present teachings provide for a mammalian expression system for production of a heterodimeric protein assembly comprising a polypeptide comprising amino acid residues 18 through 333 of the human IL1-R1 fused with IgG-Fc domain (Fc-II) via a flexible linker, and another polypeptide comprising amino acid residues 21 through 358 of the human IL-1RAcP fused with another IgG-Fc domain (Fc-V) via a flexible linker.

In an example embodiment, the mammalian expression system of the present invention comprises Chinese hamster ovary cells (CHO-K1) harboring a plasmid comprising nucleotide sequence of SEQ ID NO. 7.

### EXAMPLES

The following Examples illustrate the forgoing aspects and other aspects of the present teachings. These non-limiting Examples are put forth so as to provide those of ordinary skill in the art with illustrative embodiments as to how the compounds, compositions, articles, devices, and/or methods claimed herein are made and evaluated.

Efforts have been made to ensure accuracy with respect to numbers (e.g., amounts, temperature, etc.) but some errors and deviations should be accounted for.

**Example 1:** Construction of plasmids for expression of polypeptides of the present invention.

Optimized gene sequences comprising sequences encoding residues amino acid residues 1 through 333 of IL-1R1 or residues 1 through 358 of IL-1RAcP were chemically synthesized. The resulting fragments were cloned in-frame into the recipient intermediate vectors comprising sequences coding for Fc-V or Fc-II, via HindIII and BspEI restriction sites. Obtained positive clones were verified by DNA sequencing. The resulting constructions were termed PKN001'-IL-1R-FcV and PKN001'-RAcP-FcII, respectively.

The gene encoding IL-1R1-Fc-V was then cloned into a second intermediate vector, termed PKN002, via HindIII and EcoRI restriction sites. Positive clones were screened by double digestion and the insertion sequence of the correct plasmids was verified by DNA sequencing. The resulting constructions were termed PKN002-IL-1R-FcV.

Finally, the expression cassette for RAcP-FcII from PKN001 '-RAcP-FcII was integrated into PKN002-II,-1R-FcV plasmid via NotI and SalI restriction sites and yielded a new recombinant construction that contained expression elements for both RAcP-FcII and IL-1R-FcV. The resulting clones were screened by NotI and SalI double digestion followed by DNA gel electrophoresis, the correct clones were exhibiting one band migrating approximately as an 8kb DNA fragment and another band migrating approximately as a 4kb DNA fragment. The final plasmid was termed PKN012-IL1R-FcV-RAcP-FcII.

Recombinant plasmid PKN012-IL1R-FcV-RAcP-FcII combines expression cassettes for both RAcP-FcII and IL-1R-FcV. The plasmid can be used for co-expressing RAcP-FcII and IL-1R-FcV proteins in a 1 to 1 ratio under the control of a CMV promoter. The majority of these two fusion proteins would then form IL1R-FcV/RAcP-FcII heterodimers after secretion. The plasmid also expresses Glutamine synthetase (GS) protein via a SV40 promoter, which can be used as a selection marker to generate stable cell lines for IL1R-FcV/RAcP-FcII heterodimer production. The plasmid map for PKN012-II,1R-FcV-RAcP-FcII is illustratively shown in Figure 2.

**Example 2:** Generation of stable cell lines for expressing polypeptides of the present invention.

A stable clone of CHO-K1 cells co-expressing hIL1-R1-hIgG1-Fc polypeptide of SEQ ID NO. 1 and hIL-IRAcP-hIgGI-Fc polypeptide of SEQ ID NO. 2 has been generated through standard cell biology protocols. The expression plasmid PKN012-IL1R-FcV-RAcP-FcII described in Example 1 was used for generating stable cell lines for high expression of said polypeptides. Expression levels of said polypeptides in a plurality of clones were about or over 100 mg/L in a 7-day batch culture. One clonal cell line showed expression levels of about or over 300 mg/L in shake flask batch culture.

### Materials

Chinese hamster ovary cells (CHO-K1) were obtained as frozen stocks from ATCC (CCL-61^{™}). The cells were adapted in house into a CD CHO media. Media and reagent were obtained from commercial source. Upon full adaptation, the cells were grown to high density for a few passages. The resulting cells were subcloned. One of the resulting clones with a doubling time under 20 hrs and good morphology was selected as parental cell line.

### Methods

CHO-K1 cells at passage 4 were cultured in CD-CHO chemically defined media (Invitrogen) containing 6 mM L-Glutamine. The cells were maintained by 1:3 splits after reaching a cell density of 4×10⁶ cells/ml. Cells were span down by centrifugation and resuspended into 1 ml of CD-CHO chemically defined media (Invitrogen).

The following electroporation protocol was utilized:
- 40 ug of plasmid DNA in 100 ul sterilized TE buffer was used for an electroporation; on the day of transfection the viability of cells was at least 95%;
- cells were centrifuged at 800rpm for 5 min; the super was removed and the cells were resuspended in 10 ml CD CHO media and centrifuged again;
- the super was removed and the cells were resuspended in a small vol. of CD CHO media to 1.43×10⁷ cells/ml;
- 0.7 ml cells (10⁷ cells) were added to the DNA and mixed gently by pipetting, avoiding generating bubbles;
- cells were immediately electroporated by delivering a single pulse of 300 volts, 900 uF to each cuvette;
- 50 ml CD CHO (without L-glutamine) was immediately added to the electroporated cells and mixed gently;
- the cell suspension was distributed into ten 96 well plates at 50 ul/well; the following day, 150 ul of CD CHO containing 33.3 uM MSX was added to each well.

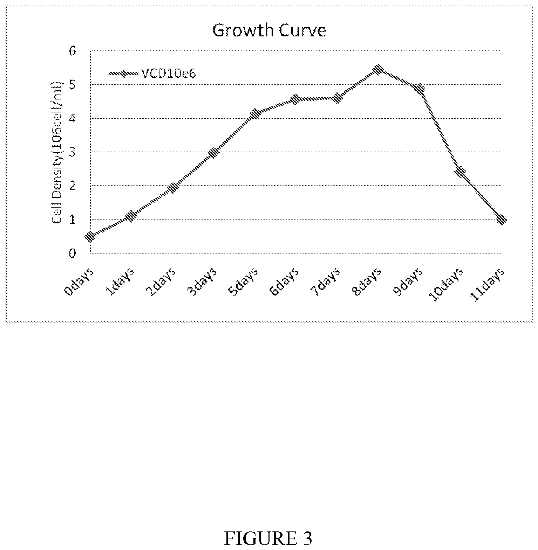

A number of transfections were carried out in CHO-K1 cells in the process of generation of potential IL1R-FcV-RAcP-FcII expression cell line; a representative transfection growth curve is shown in **Figure 3** and the data is shown in Table 1. Protein expression levels were analyzed by SDS-PAGE. Cell lines with high levels of protein overexpression exhibit a strong band with an apparent molecular weight of about 180 kDa. Based on the preliminary analysis four clones were selected for further analysis, where expression levels were further assessed by SDS-PAGE and ELISA. Well expressing clones were inoculated into 125 ml shake flasks, the cells were expanded and frozen.

The chosen highest production cell line was selected and thawed into CD-CHO. Growth curves on the cell line were assessed, and samples were collected daily for cell count, cell viability and IL1R-FcV-RAcP-FcII productivity. Based on these studies, it was determined that selected highest productivity cell line was expressing IL1R-FcV-RAcP-FcII heterodimer in CD-CHO media in amounts necessary to support commercial production. The yield of the heterodimer after purification was at least about 300 mg/L (without any production optimization).

| **Table 1:** A Representative Clonal Cell Line Growth curve | | |
|---|---|---|
| **Culture period (DAY)** | **Cell density (10⁶ cells/ml)** | **Feeding** |
| 0 | 0.5 | |
| 1 | 1.09 | |
| 2 | 1.93 | |
| 3 | 2.97 | +10% feed B |
| 5 | 4.13 | |
| 6 | 4.55 | +10% feed B |
| 7 | 4.6 | |
| 8 | 5.46 | +10% feed B |
| 9 | 4.86 | |
| 10 | 2.4 | |
| 11 | 1 | |

### Example 3: Purification of polypeptides of the present invention.

HIL1-R1-hIgG1-Fc polypeptide of SEQ ID NO. 1 and hIL-1RAcP-hIgG1-Fc polypeptide of SEQ ID NO. 2 were co-expressed in CHO-K1 essentially as described in foregoing Example 2. Cells were harvested and lysed utilizing well established protocols. After cell lysate clarification, the supernatant at protein concentration of about 0.4 mg/ml, containing expressed HIL1-R1-hIgG1-Fc/ hIL-IRAcP-hIgGI-Fc polypeptides, was applied to a Protein A affinity column. The affinity purification step was carried out according to the procedure outlined in Table 2. The Protein A eluate containing HIL1-R1-hIgG1-Fc/ hIL-1RAcP-hIgG1-Fc at pH of about 3.5 - 3.7 is incubated for 45-60 minutes to inactivate potentially existing in the contaminating materials.

After material incubation for about 45 minutes at pH 3.6 at room temperature its pH is adjusted to about 7.9-8.1 with 2 M Tris-HCl pH 9.5 (~ 3.5% v/v of the diluted Pro A eluate). The low pH treated Protein A eluate is pH-adjusted to pH of about 8.0 using 1 M Tris-HCl, pH 9.0. The conductivity is adjusted to about 5.5 mS/cm with deionized water (dH2O) if needed.

In order to reduce the contents of DNA, HCP, endotoxin and potential viral contaminants, the pH adjusted Protein A column eluate was further purified by anion-exchange chromatography (AIEX) utilizing Q Sepharose resin. The AIEX step is operated according to a step-elution procedure outlined in Table 3. Pooled elution peak fractions are concentrated by microfiltration to a concentration of about 20 mg/ml, followed by addition of a 20% Sucrose stock solution to a final Sucrose concentration of about 1% (w/v) and freezing at -80°C.

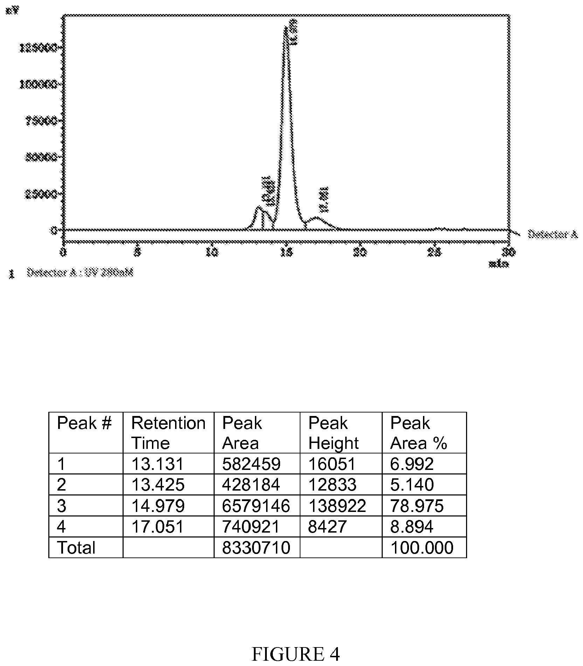

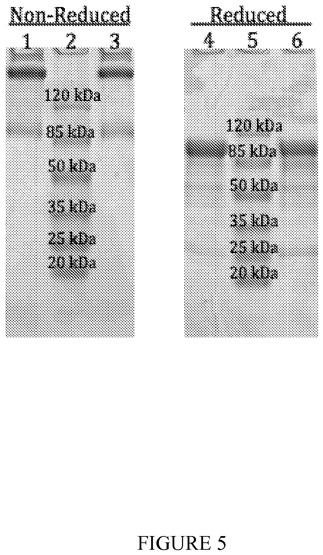

A sample of thus purified protein was analyzed by size-exclusion HPLC (SEC-HPLC) and SDS-PAGE (reducing and non-reducing). The results of the analysis are presented in Figure 4 and **Figure 5****,** respectively. In **Figure 5****,** lanes 1, 3 show hIL1-R1-hIgG1-Fc/ hIL-1RAcP-hIgG1-Fc SDS-PAGE under non-reducing conditions, while lanes 4, 6 - under reducing conditions. Lanes 2, 5 show molecular weight markers. hIL1-R1-hIgG1-Fc/ hIL-1RAcP-hIgG1-Fc heterodimer has an apparent molecular weight of about 180 kDa, consisting of two di-sulfide linked monomers, each of an apparent molecular weight of about 90 kDa.

The SEC-HPLC operational procedure is outlined in Table 4. Loading sample is diluted with mobile phase to reach protein concentration of about 5 mg/ml. The biologically relevant form of hIL1-R1-hIgG1-Fc/ hIL-IRAcP-hIgGI-Fc heterodimer is represented by the major peak with retention time RT=14.979 min.

**Table 2: The operational procedure of Protein A Affinity Chromatography**

| **Step** | **Buffer** | **Vol CV** | **Flow cm/h** |
|---|---|---|---|
| Rinse Before-use | dH2O | 3 | 150 |
| Equilibration | 10 mM NaPh, pH 6.0 | 5 | 150 |
| Sample Load | Cell harvest | | 150 |
| Wash 1 | 10 mM NaPh, pH 6.0 | 3 | 150 |
| Wash 2 | 25 mM NaPh, 0.5 MNaCl, 5% Isopropanol pH 7.0 | 5 | 150 |
| Re-Equilibration | 10 mM NaPh, pH 6.0 | 3 | 150 |
| Elution | 20 mM Na-Citrate, pH 3.4 | 4 | 150 |
| Re-Equilibration | 10 mM PB, pH 6.0 | 3 | 150 |
| CIP | 0.1 M NaOH (contact time 15 min), reversed flow, CIP every 5 cycles | 3 | 100 |
| Rinse with NaCl | 1M NaCl | 3 | 150 |
| Rinse After-use | dH2O | 3 | 150 |
| Storage | 20% (v/v) Ethanol, 20 mM NaPh, pH 7.0 | 3 | 150 |

**Table 3: The operational procedure of AIEX**

| **Step** | **Buffer** | **Vol CV** | **Flow cm/h** |
|---|---|---|---|
| Rinse Before-use | dH2O | 3 | 150 |
| Recharge | 10 mM Tris-HCl, 1 M NaCl, pH 8 | 3 | 150 |
| Equilibration | 10 mM Tris-HCl, 50 mM NaCl, pH 8 | 3 | 150 |
| Sample Load | Prepared Q Load | - | 150 |
| Wash | 10 mM Tris-HCl, 50 mM NaCl, pH 8 | 3 | 150 |
| Elution | 10 mM Tris-HCl, 0.35 M NaCl, pH 8.0 | 3 | 150 |
| CIP | 1 M NaOH (contact time 1 hr), reversed-flow | 3 | 40 |
| Regeneration | 10 mM Tris-HCl, 1 M NaCl, pH 8.0 | 3 | 150 |
| Rinse After-use | dH2O | 3 | 150 |
| Storage | 20% (v/v) Ethanol | 3 | 150 |

**Table 4: The Operational Procedure of SEC-HPLC**

| | |
|---|---|
| Mobile Phase: | 20 mM phosphate, 300 mM NaCl, pH 7.4 |
| Flow Rate: | 0.5 mL/min |
| Column: | G2000 SWxl, 7.8mm×300mm, TOSOH Bioscience |
| Guard column: | TSK Guard SWxl,6.0mm×40mm, TOSOH Bioscience |
| Column Temperature: | 25 °C |
| Sampler temperature: | Rome temperature |
| Injection Volume: | 10 µl |
| Detector Wavelength: | 280 nm |
| Run Time: | 30 min |

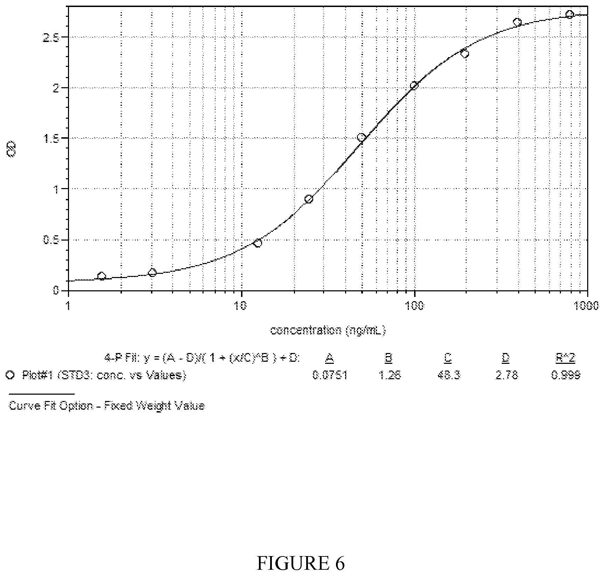

The activity of thus purified sample, which was expressed and purified essentially as described in this example, was tested in a standard ELISA assay using commercially available human IL-1β (PrimGene, Shanghai, China; Cat#: 101-01B). A typical binding curve obtained in the assay is shown in **Figure 6****.** Based on the curve analysis, the calculated EC50 value is about 50 ng/ml.

**Example 4:** Characterization of inhibition of IL-1β-induced IL-6 production with IL1R-FcV-RAcP-FcII heterodimer in human cells.

The present Example demonstrates potent functional (inhibitory) properties of IL1R-FcV-RAcP-FcII heterodimer in targeting human IL-1β. As a functional comparator, previously characterized mouse monoclonal antibodies against IL-1β were used. Human lung fibroblasts MRC5 produce IL-6 in response to treatment with IL-1β. Quantification of IL-1β-induced IL-6 production in MRC5 cells was used as the assay functional output. Inhibition of IL-6 production by IL1R-FcV-RAcP-FcII heterodimer and by control anti IL-1β antibody provides a quantitative measure of inhibitory properties of the IL1R-FcV-RAcP-FcII heterodimer preparation.

Polypeptides of II,1R-FcV-RAcP-FcΠ heterodimer (SEQ ID NO.1 and SEQ ID NO. 2) were co-expressed and purified essentially as described in the forgoing examples. The protein concentration of substantially pure IL1R-FcV-RAcP-FcII heterodimer in the sample used was 0.9 mg/ml in 100 mM NaCl, 25 mM NaH₂PO₄/Na₂HPO₄, 25 mM Arginine hydrochloride, 1% Sucrose; pH=6.3.

The following materials were used in the assay:
Cells: MRC5 cells, Human Lung Fibroblasts, ATCC Cat # CCL-171, Lot # 59474707.
Medium: DMEM, Dulbecco's Modification of Eagle's Medium, high glucose (4.5 g/L), Mediatech, Cat # 10-017-CM, Lot # 10017204, supplemented with L-glutamine and 1x penn/strep and 10% Fetal Bovine Serum, Omega Scientific, Cat # FB-05, Lot # 105104
Reagents: IL-1β, Human recombinant, E. coli-derived, Ala1 17-Ser269, Accession # NP_000567, R&D systems, Cat # 201-LB, Lot # ADM1411062; Monoclonal antibodies against human IL-1β, clone #8516, R&D systems, Cat # MAB201, Lot # AWE1011081;
Human IL-6 Quantakine Immunoassay, R&D systems, Cat # D6050, Lot # 300070.

The following procedures were utilized in the assay:

### Cell Maintenance:

1. Propagate MRC5 cells in the DMEM containing 10% FBS on T75 flasks according to the manufacturer's recommendations. Record the passage number;
2. Trypsinize cells, re-suspend in DMEM containing 10% FBS;
3. Test cell viability using Guava ViaCount Reagent. Standard viability should be >85%, if less, do not use this batch of cells;
4. Prepare dilutions of the cells for plating at desired density of 2 × 103 cells/well per well of 48-well plate or 5 × 103 per ml;
5. Allow cells to attach for 16-24 hours at 37°C, 5% CO₂, then remove medium;
6. Replace with fresh DMEM medium supplemented with 10% FBS, 0.4 ml/well for 48-well plate, incubate 5-10 min and replace the medium again with the same volume;
7. Add to the wells IL-1β, Test Substances pre-mixed with IL-1β and appropriate controls;
8. Incubate the cells after treatment for 24 hours then harvest the supernatants;
9. Centrifuge the supernatants at 300 × g for 10 min, collect cleared supernatants and use them for ELISA either directly or with 1/3 dilution if appropriate;

### ELISA Protocol:

Principle of the assay: the assay employs the quantitative sandwich enzyme immunoassay technique. A microplate is pre-coated with a monoclonal antibody specific for IL-6.

Standards and samples are pipetted into the wells of the microplate and any IL-6 present binds to the immobilized antibodies. After washing away any unbound substances, an enzyme-linked polyclonal antibody specific for IL-6 is added to the wells. Following a wash to remove any unbound antibody-enzyme reagent, a substrate solution is added to the wells and color develops in proportion to the amount of bound IL-6. The color development is stopped and the color intensity is measured.

### Assay procedure:

1. Prepare all reagents and working standards as described in manufacturer's manual (http://www.rndsystems.com/product_results.aspx?k=D6050)
2. Remove excess microplate strips from the plate frame, return them to the foil pouch containing the desiccant pack, and reseal.
3. Add 100 µL of Assay Diluent RD1W to each well.
4. Add 100 µL of Standard, sample, or control per well. Cover with the adhesive strip provided. Incubate for 2 hours at room temperature. A plate layout is provided to record standards and samples assayed.
5. Aspirate each well and wash, repeating the process three times for a total of four washes. Wash by filling each well with Wash Buffer (400 µL) using a squirt bottle, manifold dispenser, or autowasher. Complete removal of liquid at each step is essential to good performance. After the last wash, remove any remaining Wash Buffer by aspirating or decanting. Invert the plate and blot it against clean paper towels.
6. Add 200 µL of IL-6 Conjugate to each well. Cover with a new adhesive strip. Incubate for 2 hours at room temperature.
7. Repeat the aspiration/wash as in step 5.
8. Add 200 µL of Substrate Solution to each well. Incubate for 20 minutes at room temperature. Protect from light.
9. Add 50 µL of Stop Solution to each well. The color in the wells should change from blue to yellow. If the color in the wells is green or the color change does not appear uniform, gently tap the plate to ensure thorough mixing.
10. Determine the optical density of each well within 30 minutes, using a microplate reader set to 450 nm. If wavelength correction is available, set to 540 nm or 570 nm. If wavelength correction is not available, subtract readings at 540 nm or 570 nm from the readings at 450 nm. This subtraction will correct for optical imperfections in the plate. Readings made directly at 450 nm without correction may be higher and less accurate.

Following are the experimental data obtained:

### Inhibition of IL-6 production by control anti-LL-1β antibodies:

1. MRC5 cells (Passage 5) were plated into a 48-well plate (Costar, Cat # 3548) at 2 × 10³ cells per well, 0.4 ml per well, and incubated for 24 hours;
2. On the day of the assay, medium from the wells was aspirated and replaced with 0.4 ml of DMEM supplemented with 10% FBS, incubated 10-15 min and replaced with the same amount of the same fresh medium again;
3. IL-1β was used at a final concentration of 50 pg/ml throughout the experiment;
4. Anti-human IL-1β antibodies were used at final concentrations of 0.192, 0.96, 4.8, 24, 120 and 600 ng/ml;
5. Both IL-1β and anti-human IL-1β antibodies were prepared as 10x concentrated solutions;
6. Prior to adding the test substances to the cells, 55 µl of 10x IL-1β was mixed with 55 µl of 10x corresponding anti-human IL-1β antibody solution (or appropriate control) and incubated at room temperature for 30 min;
7. After the incubation, 100 µl of each mixture was added to the wells containing 0.4 ml of growth medium;
8. The cells were incubated in the presence of the test substances for 24 hours and supernatants were collected, centrifuged at 300 × g for 10 min and used for IL-6 ELISA assay;
9. To test for recovery of IL-6 in the presence of anti-IL-1β antibody, the latter was used at a final concentration of 600 ng/ml and were spiked with IL-6 standard to a final concentration of 20 pg/ml.

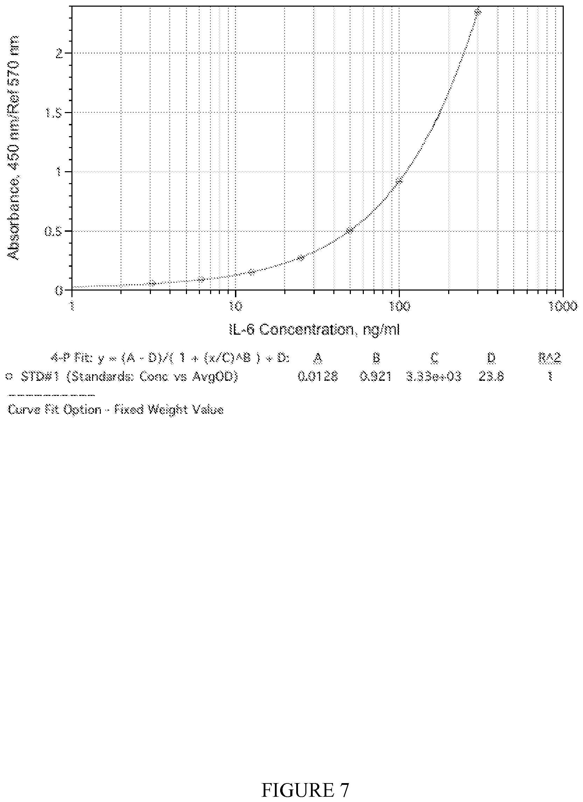

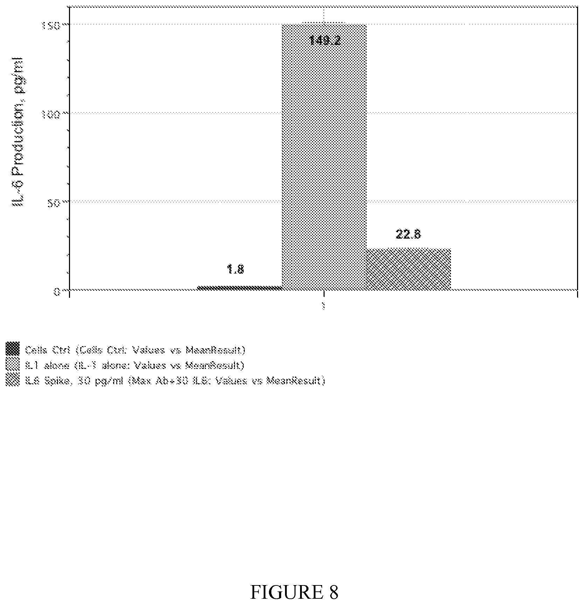

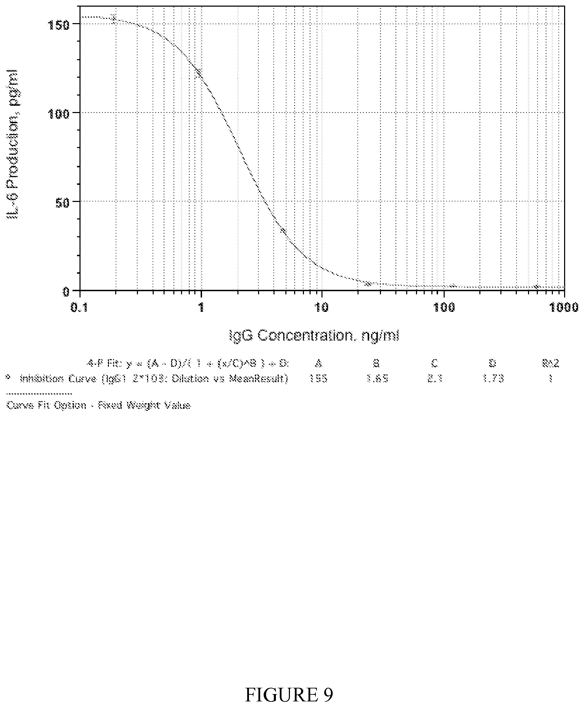

The calibration curve obtained in the IL-1 antibody mediated inhibition of IL-6 production assay is shown in **Figure 7****.** The results of the measurements of IL-1 β-mediated IL-6 production and of IL-6 recovery from the culture medium in the experiment are shown in **Figure 8** and **Figure 9****.** The obtained numerical values for IL-6 production along with their standard deviations are presented in **Table 5.**

**Table 5: Summary of IL-6 production values.**

| Sample ID | Average, pg/ml | StDev pg/ml |
|---|---|---|
| Cells Control | 1.8 | 0.001 |
| IL-1 alone, 50 pm/ml | 149.2 | 1.2 |
| Anti-IL-1 Ab + 30 pg/ml IL-6 | 22.8 | 0.2 |
| IL-1 + 0.192 ng/ml anti IL-1 Abs | 152.5 | 2.5 |
| IL-1 + 0.96 ng/ml anti IL-1 Abs | 122.1 | 2.3 |
| IL-1 + 4.8 ng/ml anti IL-1 Abs | 33.4 | 0.4 |
| IL-1 + 24 ng/ml anti IL-1 Abs | 3.37 | 0.09 |
| IL-1 + 120 ng/ml anti IL-1 Abs | 2.44 | 0.02 |
| IL-1 + 600 ng/ml anti IL-1 Abs | 2.16 | 0.03 |

The foregoing experiment revealed that: (1) the control anti-IL-1β antibody is a very potent inhibitor of IL-1β signaling pathway with an IC50 of about 2.1 ng/ml or about 14 pM; (2) plating cell density of 2 × 10³ cells per well 24 hours prior to the assay setup is adequate; (3) recovery of IL-6 spiked into the culture medium at a final concentration of 20 pg/ml is about 114%.

### Inhibition of IL-6 production by IL1R-FcV-RAcP-FeII heterodimer preparation:

1. MRC5 cells (Passage 6) were plated into 48-well plate (Costar, Cat # 3548) at 2 × 103 cells per well, 0.4 ml per well and incubated for 24 hours;
2. Plating for this experiment was carried out in duplicates. Each plating duplicate was measured in duplicates on ELISA plate resulting in 4 experimental points per treatment
3. On the day of the assay, medium from the wells was aspirated and replaced with 0.4 ml of DMEM supplemented with 10% FBS, incubated 10-15 min and replaced with the same amount of the same fresh medium again;
4. IL-1β was used at a final concentration of 50 pg/ml throughout the experiment;
5. II,1R-FcV-RAcP-FcΠ heterodimer was used at final concentrations of 0.0536, 0.134, 0.335, 0.839, 2.097, 5.24,13.1, 32.8, 81.9, 204.8, 512, 1280, 3200 and 20000 ng/ml;
6. Both IL-1β and IL1R-FcV-RAcP-FcII heterodimer was prepared as 10x concentrated solutions;
7. Prior to adding the test substances to the cells, 120 µl of 10x IL-1β/IL-1F2 was mixed with 120 µl of 10x corresponding II,1R-FcV-RAcP-FcΠ heterodimer solution (or appropriate control) and incubated at room temperature for 30 min;
8. After the incubation, 100 µl of each mixture was added to the wells containing 0.4 ml of growth medium;
9. The cells were incubated in the presence of the test substances for 24 hours and supernatants were collected, centrifuged at 300 × g for 10 min and used for IL-6 ELISA assay.

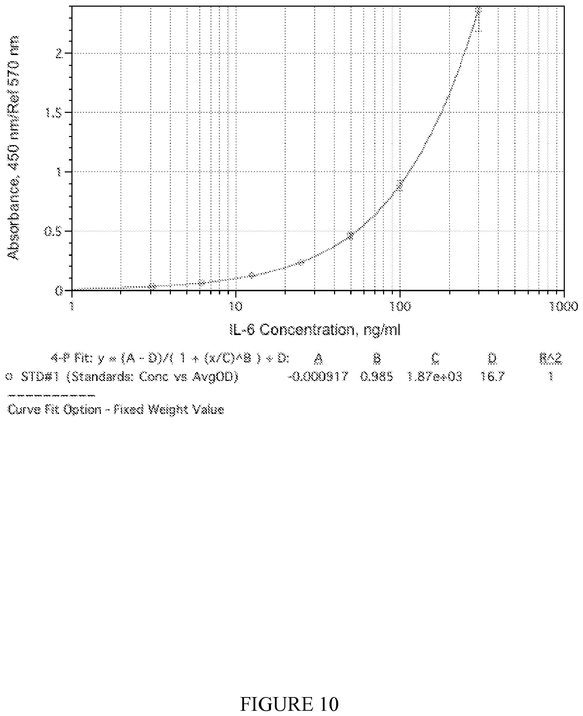

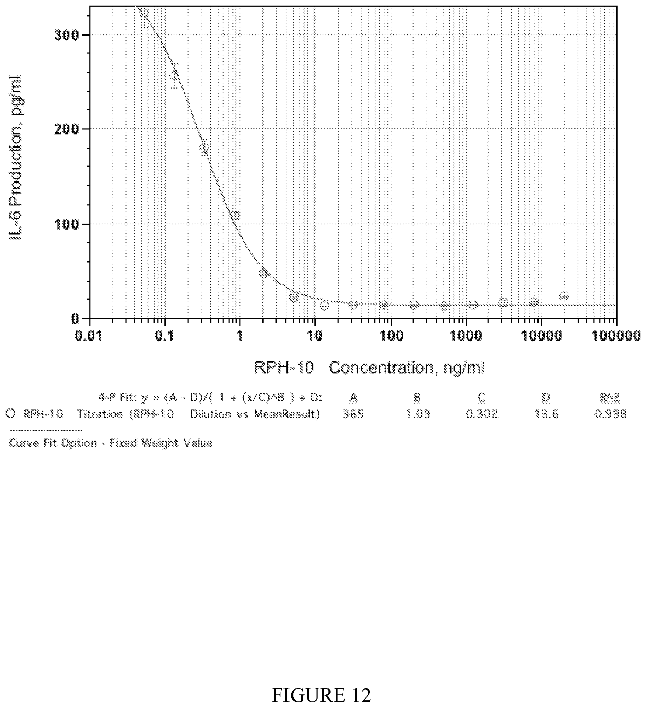

The calibration curve obtained in the II,1R-FcV-RAcP-FcΠ heterodimer mediated inhibition of IL-6 production assay is shown in **Figure 10****.** The results of the measurements of IL1R-FcV-RAcP-FcII heterodimer -mediated IL-6 production and of IL-6 recovery from the culture medium in the experiment are shown in **Figure 11** and **Figure 12****.** The obtained numerical values for IL-6 production along with their standard deviations are presented in **Table 6.**

**Table 6: Summary of IL-6 production values.**

| Sample ID | Average, pg/ml | StDev pg/ml | Calibration Range |
|---|---|---|---|
| Cells Control | 13.97 | 0.23 | |
| IL-1 alone, 50 pm/ml Plate 1 | 548.9 | 7.29 | R |
| IL-1 alone, 50 pm/ml Plate 2 | 563.1 | 1.87 | R |
| IL-1 + 0.054 ng/ml heterodimer | 322.9 | 15.69 | R |
| IL-1 + 0.134 ng/ml heterodimer | 256.2 | 12.51 | |
| IL-1 + 0.335 ng/ml heterodimer | 180.2 | 8.69 | |
| IL-1 + 0.834 ng/ml heterodimer | 108.6 | 3.86 | |
| IL-1 + 2.09 ng/ml heterodimer | 46.7 | 1.39 | |
| IL-1 + 5.24 ng/ml heterodimer | 21.9 | 1.26 | |
| IL-1 + 13.11 ng/ml heterodimer | 12.9 | 0.24 | |
| IL-1 + 32.8 ng/ml heterodimer | 13.9 | 0.29 | |
| IL-1 + 81.92 ng/ml heterodimer | 13.4 | 1.04 | |
| IL-1 + 204.8 ng/ml heterodimer Pl.1 | 13.4 | 0.64 | |
| IL-1 + 512 ng/ml heterodimer | 12.9 | 0.65 | |
| IL-1 + 1280 ng/ml heterodimer | 13.9 | 0.16 | |
| IL-1 + 3200 ng/ml heterodimer | 16.1 | 3.03 | |
| IL-1 + 8000 ng/ml heterodimer | 16.9 | 1.09 | |
| IL-1 + 20000 ng/ml heterodimer | 22.5 | 0.55 | |
| heterodimer alone 20000 ng/ml | 20.9 | 1.04 | |
| IL-1 + 204.8 ng/ml heterodimer Pl. 2 | 13.9 | 0.68 | |
| heterodimer + 30 pg/ml IL-6 Spike | 27.4 | 1.2 | |

| | | | |
|---|---|---|---|
| R - indicates that the value fell outside the assay calibration range | | | |

Each experimental point was plated in duplicate and IL-6 production was further measured in duplicate resulting in 4 experimental reads per concentration point. The obtained data demonstrated high reproducibility of the experimental procedure and the assay. The foregoing experiment revealed that the IC50 value for IL1R-FcV-RAcP-FcII heterodimer in this experiment was about 0.3 mg/ml or about 2.4 pM.

**Example 5:** Pharmacokinetics (PK) of IL1R-FcV-RAcP-FcII heterodimer after subcutaneous administration in mice.

Polypeptides of II,1R-FcV-RAcP-FcΠ heterodimer (SEQ ID NO.1 and SEQ ID NO. 2) were co-expressed and purified essentially as described in the forgoing examples. For administration into animals, the polypeptides were formulated in the following buffer: 1% w/v Sucrose, 100mM Sodium Chloride, 20 mM L-Arginine Hydrochloride, 25 mM Sodium Bicarbonate, pH 6.3. The dosing stock concentration used was 0.5 mg/mL of the polypeptide.

Fourteen female Balb/c nu/nu mice were randomized based on body weight into seven groups of two animals on Day 0 of the study. A single treatment of the polypeptides (5 mg/kg) was administered subcutaneously (dorsal) on Day 0 to all groups except mice in Group 1, which were bled via cardiac puncture for plasma preparation on Day 0 of the study. Blood samples were collected from mice via the orbital sinus in the remaining groups at various times throughout the study for preparation of plasma.

Body weights were recorded for all animals on the treatment day (Day 0) and then three times per week, including the termination day of each group.

Groups of mice were culled at specific time points for plasma preparation. Body weight changes were not measured in groups culled for sample collection at 0 hours and within 36 hours of dose administration. All other mice gained body weight and no adverse clinical signs were reported during the study period.

Following the in-life phase of the study, plasma samples were analyzed by ELISA for Hu-Fc proteins. Quantification of Hu-Fc in mouse plasma samples by ELISA was used as a read-out for circulating levels of the polypeptides. The assay was performed on samples from all mice in the study.

The polypeptides were detected in the plasma of animals at 1 hour post-administration. One Phase Decay Model equation using Prism 5.0c (GraphPad Software Inc, La Jolla, CA, USA) was then used to determine pharmacokinetics of the polypeptides as detected by Hu-Fc ELISA. Peak circulating level of Hu-Fc (Cmax) was determined to be 1.65 µg/mL, and time to peak circulating levels (Tmax) was 36 hours post treatment. The half-life (T1/2) was 88.15 hours and the rate constant (K) was 0.0079 hr-1. Hu-Fc levels were negligible in the plasma of the untreated Group 1 animals. The results of the study are summarized in Table 7.

**Table 7: Mean Human-Fc Protein Concentration ± SEM (µg/mL) at each Time Post-Administration**

| Group | Treatment | Bleeding Schedule (time post-administration) | Mean Human-Fc Protein Concentration [µg/mL] | SEM |
|---|---|---|---|---|
| 1 | No treatment | 0 hours | 0.00 | -⁻* |
| 2 | polypeptide of SEQ IDs NO. 1 and NO. 2 (5 mg/kg, Once only, s.c.) | 30 minutes | 0.02 | 0.01 |
| 3 | | 1 hour^ | 0.12 | 0.00 |
| 4 | | 2 hours^ | 0.24 | 0.09 |
| 5 | | 4 hours^ | 0.76 | 0.03 |
| 6 | | 8 hours^ | 1.11 | 0.10 |
| 7 | | 10 hours^ | 1.53 | 0.08 |
| 2 | | 24 hours^{#} | 1.47 | 0.14 |
| 3 | | 36 hours^{#} | 1.65 | 0.11 |
| 4 | | 96 hours^{#} | 1.27 | 0.01 |
| 5 | | 7 days^{#} | 0.43 | 0.01 |
| 6 | | 14 days^{#} | 0.13 | 0.07 |
| 7 | | 21 days^{#} | 0.06 | 0.04 |

| | | | | |
|---|---|---|---|---|
| *SEM unable to be calculated as level of Hu-Fc was below detectable limit of ELISA for one of samples in group. The Human-Fc Protein Concentration was determined by Prism Software based on the mean absorbance of the triplicate samples Bleed via orbital sinus ^{#} Bleed via terminal cardiac puncture | | | | |

**Example 6:** Evaluation of the toxicity of II,1R-FcV-RAcP-FcΠ heterodimer following 28-days repeated dosing in C57BL6 mice.

Following is a repeat dose murine toxicity evaluation study results summary. Treatment of C57BL6 mice with polypeptides of II,1R-FcV-RAcP-FcΠ heterodimer (SEQ ID NO.1 and SEQ ID NO. 2) test article by twice weekly subcutaneous injection at 10, 30 and 100 mg/kg dose levels was well tolerated in the study. There were no unscheduled deaths prior to completion of the study. Test article treatment was not associated with morbidity or clinical signs of toxicity. There was no consistent gender or treatment related effect on body weight. There was a finding in increased food consumption which was considered to be a possible effect of treatment but this was not considered to be adverse.

Although there was evidence of functional changes in parameters related to clinical biochemistry, these were not considered to be of toxicological significance as there was evidence of reversibility in most of these parameters, although not complete in some. In the absence of toxicokinetic evaluation it was not possible to assess the significance of apparent non-reversibility of some of these differences. However, none of the differences was associated with histopathological change or was considered to be adverse. The differences observed were of a type that is typically reversible and it is possible that the reversibility period was no sufficient to demonstrate this.

It was concluded that the No-Observed Adverse Effect Level (NOAEL) in the mouse 28-day IL1R-FcV-RAcP-FcII heterodimer dosing study was therefore considered to be 100 mg/kg.

The full scope of the invention should be determined by reference to the claims and the specification.

## Claims

1. A heterodimeric protein composition capable of binding human IL-1β, said protein composition comprising:
a first polypeptide comprising
a first amino acid sequence comprising amino acids 18 through 333 of human IL1 -R 1 , and
a second amino acid sequence comprising a first mutant of a Fc portion of human immunoglobulin gamma-1 Fc;
a second polypeptide comprising
another first amino acid sequence comprising amino acids 21 through 358 of human ILl-RAcP, and
another second amino acid sequence comprising a second mutant of the Fc portion of human immunoglobulin gamma-1 Fc; and
wherein said first and second mutants are selected as to favor heterodimeric assembly between said first and second mutants over any homodimeric assembly,
wherein the first polypeptide comprises the amino acid sequence of SEQ ID NO. 1 and the second polypeptide comprises the amino acid sequence of SEQ ID NO. 2.

2. The protein composition of claim 1, wherein said protein composition exhibits human IL- 1β binding activity in an ELISA assay with an EC50 of 50 ng/ml.

3. A therapeutic composition, the composition comprising a heterodimeric protein composition capable of binding human IL-1β, said heterodimeric protein composition comprising:
a first polypeptide comprising
a first amino acid sequence comprising amino acids 18 through 333 of human IL1 -R 1, and
a second amino acid sequence comprising a first mutant of a Fc portion of human immunoglobulin gamma-1 Fc;
a second polypeptide comprising
another first amino acid sequence comprising amino acids 21 through 358 of human ILl-RAcP, and
another second amino acid sequence comprising a second mutant of the Fc portion of human immunoglobulin gamma-1 Fc; and
wherein said first and second mutants are selected as to favor heterodimeric assembly between said first and second mutants over any homodimeric assembly,
wherein the first polypeptide comprises the amino acid sequence of SEQ ID NO. 1 and the second polypeptide comprises the amino acid sequence of SEQ ID NO. 2.

4. The therapeutic composition of claim 3, wherein half-life of said heterodimeric protein composition in systemic circulation in mice after a subcutaneous administration at a dose of 5 mg/kg is at least 88 hours, as assayed by human Fc ELISA.

5. An isolated nucleic acid encoding a polypeptide comprising an amino acid sequence of any one of SEQ ID. 1, SEQ ID NO. 2, SEQ ID NO. 3, and SEQ ID NO. 5.

6. The nucleic acid of claim 5, wherein the codon usage is optimized for high expression of said polypeptide in a mammalian cell.

7. The nucleic acid of claim 5, wherein the nucleic acid sequence encodes a polypeptide comprising amino acid sequence of SEQ ID NO. 3 and comprises the sequence of SEQ ID NO. 4, or wherein the nucleic acid sequence encodes a polypeptide comprising amino acid sequence of SEQ ID NO. 5 and comprises the sequence of SEQ ID NO. 6.

8. The nucleic acid of claim 7, wherein said nucleic acid comprises an expression vector.

9. An isolated nucleic acid of SEQ ID NO. 7.

10. A heterologous expression system, the expression system harboring an expression vector comprising a nucleic acid sequence encoding a first polypeptide comprising amino acid sequence of SEQ ID NO. 3 and another nucleic acid sequence encoding a second polypeptide comprising amino acid sequence of SEQ ID NO. 5.

11. The expression system of claim 10, wherein said expression vector is harbored in a mammalian cell, preferably wherein said mammalian cell is a CHO cell.

12. The expression system of claim 11, wherein said expression system is capable of expressing a heterodimeric protein comprising a first polypeptide comprising amino acid sequence of SEQ ID NO. 1 and a second polypeptide comprising amino acid sequence of SEQ ID NO. 2, and wherein the level of expression of said heterodimeric protein is at least 300 mg per liter of cell culture.

13. A heterodimeric protein comprising a first polypeptide comprising amino acid sequence of SEQ ID NO. 1 and a second polypeptide comprising amino acid sequence of SEQ ID NO. 2 for use in the treatment or prevention of a disease associated with modulation of activity of human IL-1β, wherein said disease is an arthritis, or wherein said disease is a gout, or wherein said disease is a rheumatoid arthritis, or wherein said disease is a Cryopyrin-Associated Periodic Syndromes (CAPS), or wherein said disease is a scleroderma, or wherein said disease is a diabetes, or wherein said disease is atherosclerosis, or wherein said disease is a dry eye disease, or wherein said disease is an ocular allergy, or wherein said disease is an uveitis.

14. A heterodimeric protein composition capable of binding human IL-1β, said protein composition being capable of inhibiting human IL-6 production in human lung fibroblasts in response to treating the fibroblasts with human IL-1β, said inhibiting having an IC50 value of 2.3 pM, wherein the heterodimeric protein composition comprises a first polypeptide comprising the amino acid sequence of SEQ ID NO. 1 and a second polypeptide comprising the amino acid sequence of SEQ ID NO. 2.

## Patentansprüche

1. Heterodimere Proteinzusammensetzung, die imstande ist, humanes IL-1β zu binden, wobei die Proteinzusammensetzung umfasst:
ein erstes Polypeptid, umfassend
eine erste Aminosäuresequenz, die Aminosäuren 18 bis 333 von humanem IL1-R1 umfasst, und
eine zweite Aminosäuresequenz, die eine erste Mutante eines Fc-Abschnitts von humanem Immunglobulin gamma-1 Fc umfasst;
ein zweites Polypeptid, umfassend
eine andere erste Aminosäuresequenz, die Aminosäuren 21 bis 358 von humanem IL1-RAcP umfasst, und
eine andere zweite Aminosäuresequenz, die eine zweite Mutante des Fc-Abschnitts von humanem Immunglobulin gamma-1 Fc umfasst; und
wobei die erste und zweite Mutante so ausgewählt sind, dass sie heterodimere Anordnung zwischen der ersten und zweiten Mutante gegenüber einer homodimeren Anordnung begünstigen,
wobei das erste Polypeptid die Aminosäuresequenz von SEQ ID NR. 1 umfasst und das zweite Polypeptid die Aminosäuresequenz von SEQ ID NR. 2 umfasst.

2. Proteinzusammensetzung nach Anspruch 1, wobei die Proteinzusammensetzung in einem ELISA-Test mit einem EC50 von 50 ng/ml Bindungsaktivität für humanes IL-1β zeigt.

3. Therapeutische Zusammensetzung, wobei die Zusammensetzung eine heterodimere Proteinzusammensetzung umfasst, die imstande ist, humanes IL-1β zu binden, wobei die heterodimere Proteinzusammensetzung umfasst:
ein erstes Polypeptid, umfassend
eine erste Aminosäuresequenz, die Aminosäuren 18 bis 333 von humanem IL1-R 1 umfasst, und
eine zweite Aminosäuresequenz, die eine erste Mutante eines Fc-Abschnitts von humanem Immunglobulin gamma-1 Fc umfasst;
ein zweites Polypeptid, umfassend
eine andere erste Aminosäuresequenz, die Aminosäuren 21 bis 358 von humanem IL1-RAcP umfasst, und
eine andere zweite Aminosäuresequenz, die eine zweite Mutante des Fc-Abschnitts von humanem Immunglobulin gamma-1 Fc umfasst; und
wobei die erste und zweite Mutante so ausgewählt sind, dass sie heterodimere Anordnung zwischen der ersten und zweiten Mutante gegenüber einer homodimeren Anordnung begünstigen,
wobei das erste Polypeptid die Aminosäuresequenz von SEQ ID NR. 1 umfasst und das zweite Polypeptid die Aminosäuresequenz von SEQ ID NR. 2 umfasst.

4. Therapeutische Zusammensetzung nach Anspruch 3, wobei die Halbwertszeit der heterodimeren Proteinzusammensetzung im systemischen Kreislauf bei Mäusen nach einer subkutanen Verabreichung in einer Dosis von 5 mg/kg, nach Human-Fc-ELISA getestet, mindestens 88 Stunden beträgt.

5. Isolierte Nukleinsäure, die ein Polypeptid codiert, das eine Aminosäuresequenz einer von SEQ ID NR. 1, SEQ ID NR. 2, SEQ ID NR. 3 und SEQ ID NR. 5 umfasst.

6. Nukleinsäure nach Anspruch 5, wobei die Codon-Verwendung für hohe Expression des Polypeptids in einer Säugetierzelle optimiert ist.

7. Nukleinsäure nach Anspruch 5, wobei die Nukleinsäuresequenz ein Polypeptid codiert, das die Aminosäuresequenz von SEQ ID NR. 3 umfasst und die Sequenz von SEQ ID NR. 4 umfasst, oder wobei die Nukleinsäuresequenz ein Polypeptid codiert, das die Aminosäuresequenz von SEQ ID NR. 5 umfasst und die Sequenz von SEQ ID NR. 6 umfasst.

8. Nukleinsäure nach Anspruch 7, wobei die Nukleinsäure einen Expressionsvektor umfasst.

9. Isolierte Nukleinsäure von SEQ ID NR. 7.

10. Heterologes Expressionssystem, wobei das Expressionssystem einen Expressionsvektor beherbergt, der eine Nukleinsäuresequenz, die ein erstes Polypeptid codiert, das die Aminosäuresequenz von SEQ ID NR. 3 umfasst, und eine andere Nukleinsäuresequenz umfasst, die ein zweites Polypeptid codiert, das die Aminosäuresequenz von SEQ ID NR. 5 umfasst.

11. Expressionssystem nach Anspruch 10, wobei der Expressionsvektor in einer Säugetierzelle beherbergt ist, wobei die Säugetierzelle vorzugsweise eine CHO-Zelle ist.

12. Expressionssystem nach Anspruch 11, wobei das Expressionssystem imstande ist, ein heterodimeres Protein zu exprimieren, das ein erstes Polypeptid, das die Aminosäuresequenz von SEQ ID NR. 1 umfasst, und ein zweites Polypeptid umfasst, das die Aminosäuresequenz von SEQ ID NR. 2 umfasst, und wobei das Expressionsniveau des heterodimeren Proteins mindestens 300 mg pro Liter Zellkultur beträgt.

13. Heterodimeres Protein, das ein erstes Polypeptid, das die Aminosäuresequenz von SEQ ID NR. 1 umfasst, und ein zweites Polypeptid umfasst, das die Aminosäuresequenz von SEQ ID NR. 2 umfasst, zur Verwendung bei der Behandlung oder Vorbeugung einer Erkrankung, die mit Modulation einer Aktivität von humanem IL-1β assoziiert ist, wobei die Erkrankung eine Arthritis ist, oder wobei die Erkrankung eine Gicht ist, oder wobei die Erkrankung eine rheumatische Arthritis ist, oder wobei die Erkrankung ein Cryopyrin-assoziiertes periodisches Syndrom (CAPS) ist, oder wobei die Erkrankung eine Sklerodermie ist, oder wobei die Erkrankung ein Diabetes ist, oder wobei die Erkrankung Atherosklerose ist, oder wobei die Erkrankung eine Erkrankung des trockenen Auges ist, oder wobei die Erkrankung eine Augenallergie ist, oder wobei die Erkrankung eine Uveitis ist.

14. Heterodimere Proteinzusammensetzung, die imstande ist, humanes IL-1β zu binden, wobei die Proteinzusammensetzung imstande ist, die Produktion von humanem IL-6 in humanen Lungenfibroblasten als Reaktion auf Behandlung der Fibroblasten mit humanem IL-1β zu hemmen, wobei die Hemmung einen IC50-Wert von 2,3 pM aufweist, wobei die heterodimere Proteinzusammensetzung ein erstes Polypeptid, das die Aminosäuresequenz von SEQ ID NR. 1 umfasst, und ein zweites Polypeptid umfasst, das die Aminosäuresequenz von SEQ ID NR. 2 umfasst.

## Revendications

1. Composition protéique hétérodimère apte à se lier à l'IL-1β humaine, ladite composition protéique comprenant :
un premier polypeptide comprenant
une première séquence d'acides aminés comprenant les acides aminés 18 à 333 de l'IL1-R1 humaine, et
une seconde séquence d'acides aminés comprenant un premier mutant d'une partie Fc de l'immunoglobuline gamma-1 Fc humaine ;
un seconde polypeptide comprenant
une autre première séquence d'acides aminés comprenant les acides aminés 21 à 358 de l'IL1-RAcP humaine, et
une autre seconde séquence d'acides aminés comprenant un second mutant de la partie Fc de l'immunoglobuline gamma-1 Fc humaine ; et
dans laquelle lesdits premier et second mutants sont sélectionnés de manière à favoriser un ensemble hétérodimère entre lesdits premier et second mutants par rapport à tout ensemble homodimère,
dans laquelle le premier polypeptide comprend la séquence d'acides aminés de SEQ ID NO. 1 et le second polypeptide comprend la séquence d'acides aminés de SEQ ID NO. 2.

2. Composition protéique selon la revendication 1, dans laquelle ladite composition protéique présente une activité de liaison à l'IL-1β humaine dans un test ELISA avec une EC50 de 50 ng/ml.

3. Composition thérapeutique, la composition comprenant une composition protéique hétérodimère apte à se lier à l'IL-1β humaine, ladite composition protéique hétérodimère comprenant :
un premier polypeptide comprenant
une première séquence d'acides aminés comprenant les acides aminés 18 à 333 de l'IL1-R1 humaine, et
une seconde séquence d'acides aminés comprenant un premier mutant d'une partie Fc de l'immunoglobuline gamma-1 Fc humaine ;
un seconde polypeptide comprenant
une autre première séquence d'acides aminés comprenant les acides aminés 21 à 358 de l'IL1-RAcP humaine, et
une autre seconde séquence d'acides aminés comprenant un second mutant de la partie Fc de l'immunoglobuline gamma-1 Fc humaine ; et
dans laquelle lesdits premier et second mutants sont sélectionnés de manière à favoriser un ensemble hétérodimère entre lesdits premier et second mutants par rapport à tout ensemble homodimère,
dans laquelle le premier polypeptide comprend la séquence d'acides aminés de SEQ ID NO. 1 et le second polypeptide comprend la séquence d'acides aminés de SEQ ID NO. 2.

4. Composition thérapeutique selon la revendication 3, dans laquelle la demi-vie de ladite composition protéique hétérodimère dans la circulation systémique chez les souris après une administration sous-cutanée à une dose de 5 mg/kg est d'au moins 88 heures, comme testé par ELISA Fc humaine.

5. Acide nucléique isolé codant pour un polypeptide comprenant une séquence d'acides aminés de l'une quelconque de SEQ ID NO. 1, SEQ ID NO. 2, SEQ ID NO. 3 et SEQ ID NO. 5.

6. Acide nucléique selon la revendication 5, dans lequel l'utilisation de codons est optimisée pour une expression élevée dudit polypeptide dans une cellule de mammifère.

7. Acide nucléique selon la revendication 5, dans lequel la séquence d'acides nucléiques code pour un polypeptide comprenant la séquence d'acides aminés de SEQ ID NO. 3 et comprend la séquence de SEQ ID NO. 4, ou dans lequel la séquence d'acides nucléiques code pour un polypeptide comprenant la séquence d'acides aminés de SEQ ID NO. 5 et comprend la séquence de SEQ ID NO. 6.

8. Acide nucléique selon la revendication 7, dans lequel ledit acide nucléique comprend un vecteur d'expression.

9. Acide nucléique isolé de SEQ ID NO. 7.

10. Système d'expression hétérologue, le système d'expression hébergeant un vecteur d'expression comprenant une séquence d'acides nucléiques codant pour un premier polypeptide comprenant la séquence d'acides aminés de SEQ ID NO. 3 et une autre séquence d'acides nucléiques codant pour un second polypeptide comprenant la séquence d'acides aminés de SEQ ID NO. 5.

11. Système d'expression selon la revendication 10, dans lequel ledit vecteur d'expression est hébergé dans une cellule de mammifère, de préférence dans lequel ladite cellule de mammifère est une cellule CHO.

12. Système d'expression selon la revendication 11, dans lequel ledit système d'expression est apte à exprimer une protéine hétérodimère comprenant un premier polypeptide comprenant la séquence d'acides aminés de SEQ ID NO. 1 et un second polypeptide comprenant la séquence d'acides aminés de SEQ ID NO. 2, et dans lequel le niveau d'expression de ladite protéine hétérodimère est d'au moins 300 mg par litre de culture cellulaire.

13. Protéine hétérodimère comprenant un premier polypeptide comprenant une séquence d'acides aminés de SEQ ID NO. 1 et un second polypeptide comprenant une séquence d'acides aminés de SEQ ID NO. 2 pour une utilisation dans le traitement ou la prévention d'une maladie associée à la modulation de l'activité de l'IL-1β humaine, dans laquelle ladite maladie est une arthrite, ou dans laquelle ladite maladie est la goutte, ou dans laquelle ladite maladie est une polyarthrite rhumatoïde, ou dans laquelle ladite maladie est un syndrome périodique associé à la cryopyrine (CAPS), ou dans laquelle ladite maladie est une sclérodermie, ou dans laquelle ladite maladie est un diabète, ou dans lequel ladite maladie est l'athérosclérose, ou dans laquelle ladite maladie est une sécheresse oculaire, ou dans laquelle ladite maladie est un allergie oculaire, ou dans laquelle ladite maladie est une uvéite.

14. Composition protéique hétérodimère apte à se lier à l'IL-1β humaine, ladite composition protéique étant apte à inhiber la production d'IL-6 humaine dans les fibroblastes pulmonaires humains en réponse au traitement des fibroblastes avec l'IL-1β humaine, ladite inhibition présentant une valeur IC50 de 2,3 pM, dans laquelle la composition protéique hétérodimère comprend un premier polypeptide comprenant la séquence d'acides aminés de SEQ ID NO. 1 et un second polypeptide comprenant la séquence d'acides aminés de SEQ ID NO. 2.
